(19) Europäisches Patentamt
European Patent Office
Office européen des brevets

(11) **EP 4 374 936 A1**

(12) **EUROPEAN PATENT APPLICATION**
published in accordance with Art. 153(4) EPC

(43) Date of publication:
**29.05.2024  Bulletin 2024/22**

(21) Application number: **22845330.4**

(22) Date of filing: **20.07.2022**

(51) International Patent Classification (IPC):
**A63B 24/00** (2006.01)     **G16H 20/30** (2018.01)

(52) Cooperative Patent Classification (CPC):
**A63B 24/00; G16H 10/60; G16H 20/30;
G16H 50/30**

(86) International application number:
**PCT/CN2022/106630**

(87) International publication number:
**WO 2023/001165 (26.01.2023 Gazette 2023/04)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **21.07.2021  CN 202110824968**

(71) Applicant: **Huawei Technologies Co., Ltd.
Shenzhen, Guangdong 518129 (CN)**

(72) Inventor: **YANG, Minghan
Shenzhen, Guangdong 518129 (CN)**

(74) Representative: **MERH-IP Matias Erny Reichl
Hoffmann
Patentanwälte PartG mbB
Paul-Heyse-Strasse 29
80336 München (DE)**

(54) **EXERCISE GUIDANCE METHOD AND RELATED APPARATUS**

(57)     An exercise guidance method and a related apparatus are disclosed. The method includes: An electronic device may provide a personalized exercise recommendation for a user based on obtained exercise data and health data of the user. Exercise recommendations include a taboo-type exercise recommendation and a non-taboo-type exercise recommendation. The taboo-type exercise recommendation includes an exercise recommendation that the user is prohibited from performing. The non-taboo-type exercise recommendation includes an exercise recommendation that the user is recommended to perform. The electronic device determines a taboo-type exercise recommendation when the health data of the user meets a first condition. The electronic device determines a non-taboo-type exercise recommendation when the health data of the user meets a second condition. The taboo-type exercise recommendation helps the user avoid an exercise that cannot be performed by the user. This application may provide a personalized exercise recommendation for a user and help the user avoid an exercise that cannot be performed by the user.

FIG. 4

## Description

This application claims priority to Chinese Patent Application No. 202110824968.6, filed with the China National Intellectual Property Administration on July 21, 2021 and entitled "EXERCISE GUIDANCE METHOD AND RELATED APPARATUS", which is incorporated herein by reference in its entirety.

## TECHNICAL FIELD

This application relates to the field of electronic technologies, and in particular, to an exercise guidance method and a related apparatus.

## BACKGROUND

Exercise is an optimal manner to maintain physical health and improve fitness. Exercise manners suitable for people vary with people's characteristics such as health statuses, physiques, ages, and hobbies. In addition, with a daily change of physiological indexes of human bodies, types of exercises suitable for people vary in different time periods on a day. Lack of scientific exercise guidance easily causes excessive exercise load to bodies, causing chronic injury. In addition, for some people suffering from chronic diseases, an inappropriate exercise is likely to cause a specific health risk. For example, hypoglycemia easily occurs on some diabetes patients exercising in some time periods.

Therefore, how to generate a personalized exercise scheme for a user is a subject worth studying.

## SUMMARY

Embodiments of this application provide an exercise guidance method and a related apparatus, to provide a personalized exercise recommendation for a user and help the user avoid an exercise that cannot be performed by the user.

According to a first aspect, this application provides an exercise guidance method. The method includes: An electronic device obtains first exercise data and first health data, where the first exercise data indicates an exercise status or a specified exercise target. The electronic device determines a taboo-type exercise recommendation if the first health data meets a first condition, where the taboo-type exercise recommendation indicates that a user is prohibited from performing an exercise corresponding to the first exercise data. The electronic device determines a non-taboo-type exercise recommendation if the first health data meets a second condition, where the non-taboo-type exercise recommendation indicates the user to adjust the exercise corresponding to the first exercise data.

In this embodiment of this application, the electronic device may provide a personalized exercise recommendation for the user based on obtained health data of the user in different statuses (for example, a rest state, a running state, a walking state, a swimming state, or a mountain climbing state). In this embodiment of this application, exercise recommendations include a taboo-type exercise recommendation and a non-taboo-type exercise recommendation. The taboo-type exercise recommendation includes an exercise recommendation that the user is prohibited from performing. The non-taboo-type exercise recommendation includes an exercise recommendation that the user is recommended to perform. When the health data of the user meets different conditions, the electronic device determines different exercise recommendations. The taboo-type exercise recommendation helps the user avoid an exercise that cannot be performed by the user.

With reference to the first aspect, in a possible implementation, the method further includes: The electronic device obtains second exercise data and second health data, where the second exercise data indicates the exercise status or the specified exercise target. The electronic device determines a non-taboo-type exercise recommendation if the second health data meets the second condition, where the non-taboo-type exercise recommendation indicates the user to adjust an exercise corresponding to the second exercise data. It is described herein that the electronic device may continuously obtain exercise data and health data, and continuously provide corresponding exercise recommendations. If the electronic device determines a taboo-type exercise recommendation when obtaining the first exercise data and the first health data, the electronic device may determine a non-taboo-type exercise recommendation based on the obtained second exercise data and second health data.

In a possible implementation, the method further includes: The electronic device determines a taboo-type exercise recommendation if the second health data meets the first condition, where the taboo-type exercise recommendation indicates that the user is prohibited from performing the exercise corresponding to the second exercise data. It is described herein that the electronic device may continuously obtain the exercise data and the health data, and continuously provide the corresponding exercise recommendations. If the electronic device determines a non-taboo-type exercise recommendation when obtaining the first exercise data and the first health data, the electronic device may determine a taboo-type exercise recommendation based on the obtained second exercise data and second health data.

With reference to the first aspect, in a possible implementation, after the electronic device determines the taboo-type exercise recommendation, the method further includes: The electronic device outputs, based on the taboo-type exercise recommendation, the taboo-type exercise recommendation and performs a first operation, where the first operation is used to prohibit the user from performing the exercise corresponding to the first exercise data. Herein, the electronic device may perform the corresponding first operation based on the determined taboo-type exercise recommendation. The first operation is an operation that prohibits the user from performing an exercise in the taboo-type exercise recommendation. Because the taboo-type exercise recommendation is an exercise recommendation that is determined based on the health data of the user and that cannot be performed by the user, the electronic device automatically performs the taboo-type exercise recommendation. This prevents a health problem caused by an inappropriate exercise performed by the user.

The non-taboo-type exercise recommendation is similar. The electronic device may perform the corresponding second operation based on the determined non-taboo-type exercise recommendation. The second operation is used to adjust the exercise corresponding to the first exercise data.

In a possible implementation, the first exercise data is running on a treadmill. In this case, the first operation includes stopping the treadmill. An example is provided herein. To be specific, the electronic device may perform the corresponding first operation based on the determined taboo-type exercise recommendation. The first exercise data is running on a treadmill. In this case, the taboo-type exercise recommendation determined by the electronic device is prohibiting the user from running on the treadmill, and the electronic device performs the first operation based on the taboo-type exercise recommendation, that is, stops the treadmill. Optionally, the electronic device may be the treadmill or a device that establishes a communication connection to the treadmill.

With reference to the first aspect, in a possible implementation, the taboo-type exercise recommendation further includes exercise indication information, and the exercise indication information includes one or more of location information, direction information, apparatus information, an exercise manner, and site information for performing an exercise. To be specific, the taboo-type exercise recommendation includes both an exercise that the user is prohibited from performing and an exercise recommended to be performed, and may further provide indication information for the exercise recommended to be performed, where the indication information includes various types of information indicating the user how to perform the exercise.

The non-taboo-type exercise recommendation may further include the indication information of the exercise recommended to be performed, to indicate the user how to perform an exercise in the non-taboo-type exercise recommendation.

With reference to the first aspect, in a possible implementation, before the electronic device obtains the first exercise data and the first health data, the method further includes: The electronic device is connected to a health monitoring device. The health monitoring device is configured to provide the first health data and/or the first exercise data for the electronic device. To be specific, the health monitoring device may provide the first health data, and the first exercise data is provided by the electronic device; or the health monitoring device may provide the first exercise data, and the first health data is provided by the electronic device; or both the first health data and the first exercise data are provided by the health monitoring device.

With reference to the first aspect, in a possible implementation, the electronic device and the health monitoring device are a same device.

With reference to the first aspect, in a possible implementation, the first health data includes one or more of an age, a gender, a height, a weight, a medical history, and an allergic history that are of the user and that are prestored.

In a possible implementation, the first health data is health data obtained within preset duration. Time when the electronic device obtains the first health data is first time, and a time difference between current time and the first time is within the preset duration. In this case, the electronic device determines an exercise recommendation based on the first health data. Optionally, if the time difference between the current time and the first time is beyond the preset duration, the electronic device re-obtains latest health data as the first health data.

Optionally, time when the electronic device or the health monitoring device detects the first health data is second time, and a time difference between the current time and the second time is within the preset duration. In this case, the electronic device determines the exercise recommendation based on the first health data. Optionally, if the time difference between the current time and the second time is beyond the preset duration, the electronic device or the health monitoring device re-detects latest health data as the first health data.

With reference to the first aspect, in a possible implementation, the method further includes: The electronic device obtains environment data, where the environment data includes one or more of a weather condition, air humidity, air quality, a haze degree, an indoor formaldehyde concentration, and PM2.5, and the first condition is related to the environment data. That the electronic device determines a taboo-type exercise recommendation if the first health data meets a first condition includes: The electronic device determines the taboo-type exercise recommendation if the first health data and the environment data meet the first condition. It is described herein that the environment data may also be used as a determining parameter of the taboo-type exercise recommendation. For example, when an environment is poor, the electronic device may determine a taboo-type exercise recommendation. That is, the user cannot exercise currently.

If the first health data and the environment data meet the second condition, the electronic device determines a non-taboo-type exercise recommendation.

According to a second aspect, an embodiment of this application provides an exercise guidance system, including an electronic device and a health monitoring device. The electronic device is configured to establish a connection to the health monitoring device.

The health monitoring device is configured to provide first exercise data and first health data for the electronic device, where the first exercise data indicates an exercise status or a specified exercise target.

The electronic device is further configured to determine a taboo-type exercise recommendation if the first health data meets a first condition. The taboo-type exercise recommendation indicates that a user is prohibited from performing an exercise corresponding to the first exercise data.

The electronic device is further configured to determine a non-taboo-type exercise recommendation if the first health data meets a second condition. The non-taboo-type exercise recommendation indicates the user to adjust the exercise corresponding to the first exercise data.

In this embodiment of this application, the electronic device may establish a connection to one or more health monitoring devices. The health monitoring device may provide exercise data and health data of the user for the electronic device. The electronic device provides a personalized exercise recommendation for the user based on obtained health data of the user in different statuses (for example, a rest state, a running state, a walking state, a swimming state, or a mountain climbing state). In this embodiment of this application, exercise recommendations include a taboo-type exercise recommendation and a non-taboo-type exercise recommendation. The taboo-type exercise recommendation includes an exercise recommendation that the user is prohibited from performing. The non-taboo-type exercise recommendation includes an exercise recommendation that the user is recommended to perform. When the health data of the user meets different conditions, the electronic device determines different exercise recommendations. The taboo-type exercise recommendation helps the user avoid an exercise that cannot be performed by the user.

With reference to the second aspect, in a possible implementation, the health monitoring device is further configured to provide second exercise data and second health data for the electronic device. The second exercise data indicates the exercise status or the specified exercise target. The electronic device is further configured to determine a non-taboo-type exercise recommendation if the second health data meets the second condition. The non-taboo-type exercise recommendation indicates the user to adjust an exercise corresponding to the second exercise data. It is described herein that the electronic device may continuously obtain exercise data and health data, and continuously provide corresponding exercise recommendations. If the electronic device determines a taboo-type exercise recommendation when obtaining the first exercise data and the first health data, the electronic device may determine a non-taboo-type exercise recommendation based on the obtained second exercise data and second health data.

With reference to the second aspect, in a possible implementation, the electronic device is further configured to determine a taboo-type exercise recommendation if the second health data meets the first condition. The taboo-type exercise recommendation indicates that the user is prohibited from performing the exercise corresponding to the second exercise data. It is described herein that the electronic device may continuously obtain the exercise data and the health data, and continuously provide the corresponding exercise recommendations. If the electronic device determines a non-taboo-type exercise recommendation when obtaining the first exercise data and the first health data, the electronic device may determine a taboo-type exercise recommendation based on the obtained second exercise data and second health data.

With reference to the second aspect, in a possible implementation, the electronic device is further configured to: after determining the taboo-type exercise recommendation, output the taboo-type exercise recommendation based on the taboo-type exercise recommendation and perform a first operation. The first operation is used to prohibit the user from performing the exercise corresponding to the first exercise data. Herein, the electronic device may perform the corresponding first operation based on the determined taboo-type exercise recommendation. The first operation is an operation that prohibits the user from performing an exercise in the taboo-type exercise recommendation. Because the taboo-type exercise recommendation is an exercise recommendation that is determined based on the health data of the user and that cannot be performed by the user, the electronic device automatically performs the taboo-type exercise recommendation. This prevents a health problem caused by an inappropriate exercise performed by the user.

The non-taboo-type exercise recommendation is similar. The electronic device may perform the corresponding second operation based on the determined non-taboo-type exercise recommendation. The second operation is used to adjust the exercise corresponding to the first exercise data.

With reference to the second aspect, in a possible implementation, the first exercise data is running on a treadmill. In this case, the first operation includes stopping the treadmill. An example is provided herein. To be specific, the electronic device may perform the corresponding first operation based on the determined taboo-type exercise recommendation. The first exercise data is running on a treadmill. In this case, the taboo-type exercise recommendation determined by the electronic device is prohibiting the user from running on the treadmill, and the electronic device performs the first operation based on the taboo-type exercise recommendation, that is, stops the treadmill. Optionally, the electronic device may be the treadmill or a device that establishes a communication connection to the treadmill.

With reference to the second aspect, in a possible implementation, the taboo-type exercise recommendation further includes exercise indication information, and the exercise indication information includes one or more of location information, direction information, apparatus information, an exercise manner, and site information for performing an exercise. To be specific, the taboo-type exercise recommendation includes both an exercise that the user is prohibited from performing and an exercise recommended to be performed, and may further provide indication information for the exercise recommended to be performed, where the indication information includes various types of information indicating the user how to perform the exercise.

The non-taboo-type exercise recommendation may also include the indication information of the exercise recommended to be performed, to indicate the user how to perform an exercise in the non-taboo-type exercise recommendation.

With reference to the second aspect, in a possible implementation, the first health data includes one or more of an age, a gender, a height, a weight, a medical history, and an allergic history that are of the user and that are prestored.

In a possible implementation, the first health data is health data collected within preset duration. Time when the electronic device obtains the first health data is first time, and a time difference between current time and the first time is within the preset duration. In this case, the electronic device determines an exercise recommendation based on the first health data. Optionally, if the time difference between the current time and the first time is beyond the preset duration, the electronic device re-obtains latest health data as the first health data.

Optionally, time when the electronic device or the health monitoring device detects the first health data is second time, and a time difference between the current time and the second time is within the preset duration. In this case, the electronic device determines the exercise recommendation based on the first health data. Optionally, if the time difference between the current time and the second time is beyond the preset duration, the electronic device or the health monitoring device re-detects latest health data as the first health data.

With reference to the second aspect, in a possible implementation, the electronic device is further configured to obtain environment data, where the environment data includes one or more of a weather condition, air humidity, air quality, a haze degree, an indoor formaldehyde concentration, and PM2.5, and the first condition is related to the environment data. The electronic device is further configured to determine the taboo-type exercise recommendation if the first health data and the environment data meet the first condition. It is described herein that the environment data may also be used as a determining parameter of the taboo-type exercise recommendation. For example, when an environment is poor, the electronic device may determine a taboo-type exercise recommendation. That is, the user cannot exercise currently.

If the first health data and the environment data meet the second condition, the electronic device determines a non-taboo-type exercise recommendation.

With reference to the second aspect, in a possible implementation, the system further includes an output device. After determining the taboo-type exercise recommendation if the first health data meets the first condition, the electronic device is further configured to indicate the output device to output the taboo-type exercise recommendation, and the output device is configured to output the taboo-type exercise recommendation. After determining the non-taboo-type exercise recommendation if the first health data meets the second condition, the electronic device is further configured to indicate the output device to output the non-taboo-type exercise recommendation, and the output device is configured to output the non-taboo-type exercise recommendation. It is described herein that the electronic device may be used as a processing device, and then output the determined taboo-type exercise recommendation and non-taboo-type exercise recommendation through another device (output device).

With reference to the second aspect, in a possible implementation, the system further includes an execution device. After determining the taboo-type exercise recommendation if the first health data meets the first condition, the electronic device is further configured to indicate, based on the taboo-type exercise recommendation, the execution device to perform the first operation, and the execution device is configured to perform the first operation. After determining the non-taboo-type exercise recommendation if the first health data meets the second condition, the electronic device is further configured to indicate, based on the non-taboo-type exercise recommendation, the execution device to output a second operation, and the execution device is configured to perform the second operation. It is described herein that the electronic device may be used as a processing device, and then perform an operation corresponding to an exercise recommendation through another device (execution device). To be specific, the electronic device performs the first operation corresponding to the taboo-type exercise recommendation and the second operation corresponding to the non-taboo-type exercise recommendation through the execution device.

According to a third aspect, an embodiment of this application provides an electronic device, including an obtaining unit and a determining unit.

The obtaining unit is configured to obtain first exercise data and first health data. The first exercise data indicates an exercise status or a specified exercise target.

The determining unit is configured to determine a taboo-type exercise recommendation if the first health data meets a first condition. The taboo-type exercise recommendation indicates that a user is prohibited from performing an exercise corresponding to the first exercise data.

The determining unit is further configured to determine a non-taboo-type exercise recommendation if the first health data meets a second condition. The non-taboo-type exercise recommendation indicates the user to adjust the exercise corresponding to the first exercise data.

In this embodiment of this application, the electronic device may provide a personalized exercise recommendation for the user based on health data that is of the user in different statuses (for example, a rest state, a running state, a walking state, a swimming state, or a mountain climbing state) and that is obtained through the obtaining unit. In this embodiment of this application, exercise recommendations include a taboo-type exercise recommendation and a non-taboo-type exercise recommendation. The taboo-type exercise recommendation includes an exercise recommendation that the user is prohibited from performing. The non-taboo-type exercise recommendation includes an exercise recommendation that the user is recommended to perform. When the health data of the user meets different conditions, the electronic device determines different exercise recommendations through the determining unit. The taboo-type exercise recommendation helps the user avoid an exercise that cannot be performed by the user.

With reference to the third aspect, in a possible implementation, the obtaining unit is further configured to obtain second exercise data and second health data. The second exercise data indicates the exercise status or the specified exercise target. The determining unit is further configured to determine a non-taboo-type exercise recommendation if the second health data meets a second condition. The non-taboo-type exercise recommendation indicates the user to adjust an exercise corresponding to the second exercise data. It is described herein that the electronic device may continuously obtain exercise data and health data through the obtaining unit, and continuously provide corresponding exercise recommendations. If the electronic device determines a taboo-type exercise recommendation when obtaining the first exercise data and the first health data, the electronic device may subsequently determine, through the determining unit, a non-taboo-type exercise recommendation based on the obtained second exercise data and second health data.

In a possible implementation, the determining unit is further configured to determine a taboo-type exercise recommendation if the second health data meets the first condition. The taboo-type exercise recommendation indicates that the user is prohibited from performing the exercise corresponding to the second exercise data. It is described herein that the electronic device may continuously obtain the exercise data and the health data, and continuously provide the corresponding exercise recommendations. If the electronic device determines a non-taboo-type exercise recommendation when obtaining the first exercise data and the first health data, the electronic device may subsequently determine, through the determining unit, a taboo-type exercise recommendation based on the obtained second exercise data and second health data.

With reference to the third aspect, in a possible implementation, the electronic device further includes an output unit and an execution unit. The output unit is configured to: after the determining unit determines the taboo-type exercise recommendation, output the taboo-type exercise recommendation based on the taboo-type exercise recommendation. The execution unit is configured to perform a first operation. The first operation is used to prohibit the user from performing the exercise corresponding to the first exercise data. Herein, the electronic device may perform, through the output unit or the execution unit, the corresponding first operation based on the determined taboo-type exercise recommendation. The first operation is an operation that prohibits the user from performing an exercise in the taboo-type exercise recommendation. Because the taboo-type exercise recommendation is an exercise recommendation that is determined based on the health data of the user and that cannot be performed by the user, the electronic device automatically performs the taboo-type exercise recommendation. This prevents a health problem caused by an inappropriate exercise performed by the user.

The non-taboo-type exercise recommendation is similar. The electronic device may perform, based on the non-taboo-type exercise recommendation determined by the determining unit, a corresponding second operation through the output unit or the execution unit. The second operation is used to adjust the exercise corresponding to the first exercise data.

In a possible implementation, the first exercise data is running on a treadmill. In this case, the first operation includes stopping the treadmill. An example is provided herein. To be specific, the electronic device may perform the corresponding first operation based on the determined taboo-type exercise recommendation. The first exercise data is running on a treadmill. In this case, the taboo-type exercise recommendation determined by the electronic device is prohibiting the user from running on the treadmill, and the electronic device performs the first operation based on the taboo-type exercise recommendation, that is, stops the treadmill. Optionally, the electronic device may be the treadmill or a device that establishes a communication connection to the treadmill.

With reference to the third aspect, in a possible implementation, the taboo-type exercise recommendation further includes exercise indication information, and the exercise indication information includes one or more of location information, direction information, apparatus information, an exercise manner, and site information for performing an exercise. To be specific, the taboo-type exercise recommendation includes both an exercise that the user is prohibited from performing and an exercise recommended to be performed, and may further provide indication information for the exercise recommended to be performed, where the indication information includes various types of information indicating the user how to perform the exercise.

The non-taboo-type exercise recommendation may also include the indication information of the exercise recommended to be performed, to indicate the user how to perform an exercise in the non-taboo-type exercise recommendation.

With reference to the third aspect, in a possible implementation, the electronic device further includes a connection unit. The connection unit is configured to connect to a health monitoring device before the obtaining unit obtains the first exercise data and the first health data, and the health monitoring device is configured to provide the first health data and/or the first exercise data for the electronic device. To be specific, the health monitoring device may provide the first health data, and the first exercise data is provided by the electronic device; or the health monitoring device may provide the first exercise data, and the first health data is provided by the electronic device; or both the first health data and the first exercise data are provided by the health monitoring device.

With reference to the third aspect, in a possible implementation, the electronic device and the health monitoring device are a same device.

With reference to the third aspect, in a possible implementation, the first health data includes one or more of an age, a gender, a height, a weight, a medical history, and an allergic history that are of the user and that are prestored.

In a possible implementation, the first health data is health data obtained within preset duration. Time when the electronic device obtains the first health data is first time, and a time difference between current time and the first time is within the preset duration. In this case, the electronic device determines the exercise recommendation based on the first health data. Optionally, if the time difference between the current time and the first time is beyond the preset duration, the electronic device re-obtains latest health data as the first health data.

Optionally, time when the electronic device or the health monitoring device detects the first health data is second time, and a time difference between the current time and the second time is within the preset duration. In this case, the electronic device determines an exercise recommendation based on the first health data. Optionally, if the time difference between the current time and the second time is beyond the preset duration, the electronic device or the health monitoring device re-detects latest health data as the first health data.

With reference to the third aspect, in a possible implementation, the obtaining unit is further configured to obtain environment data, where the environment data includes one or more of a weather condition, air humidity, air quality, a haze degree, an indoor formaldehyde concentration, and PM2.5, and the first condition is related to the environment data. The determining unit is further configured to determine the taboo-type exercise recommendation if the first health data and the environment data meet the first condition. It is described herein that the environment data may also be used as a determining parameter of the taboo-type exercise recommendation. For example, when an environment is poor, the electronic device may determine, through the determining unit, a taboo-type exercise recommendation. That is, the user cannot exercise currently.

The determining unit is further configured to determine the non-taboo-type exercise recommendation if the first health data and the environment data meet the second condition.

According to a fourth aspect, this application provides an electronic device, including one or more processors and one or more memories. The memories are coupled to the processors. The memories are configured to store computer program code, and the computer program code includes computer instructions. When the computer instructions are run on the processor, the electronic device is enabled to perform the exercise guidance method according to any possible implementation of any one of the foregoing aspects.

According to a fifth aspect, an embodiment of this application provides a computer storage medium, including computer instructions. When the computer instructions are run on an electronic device, a communication apparatus is enabled to perform the exercise guidance method according to any possible implementations of any one of the foregoing aspects.

According to a sixth aspect, an embodiment of this application provides a computer program product. When the computer program product is run on a computer, the computer is enabled to perform the exercise guidance method according to any possible implementations of any one of the foregoing aspects.

## BRIEF DESCRIPTION OF DRAWINGS

FIG. 1 is a schematic diagram of a system architecture according to an embodiment of this application;
FIG. 2 is a schematic diagram of a structure of an electronic device according to an embodiment of this application;
FIG. 3 is a schematic diagram of a structure of a health monitoring device according to an embodiment of this application;

FIG. 4 is a method flowchart of an exercise guidance method according to an embodiment of this application;

FIG. 5a and FIG. 5b are diagrams of application scenarios of an exercise guidance method according to an embodiment of this application;

FIG. 6a to FIG. 6d are schematic diagrams of a group of application interfaces according to an embodiment of this application;

FIG. 7a to FIG. 7d are schematic diagrams of another group of application interfaces according to an embodiment of this application; and

FIG. 8 is a schematic diagram of software modules of an electronic device according to an embodiment of this application.

## DESCRIPTION OF EMBODIMENTS

The following describes technical solutions in embodiments of this application with reference to accompanying drawings. In descriptions of embodiments of this application, "/" means "or" unless otherwise specified. For example, A/B may represent A or B. In this specification, "and/or" describes only an association relationship of associated objects and represents that three relationships may exist. For example, A and/or B may represent the following three cases: Only A exists, both A and B exist, and only B exists. In addition, in the descriptions of embodiments of this application, "a plurality of" means two or more.

The following terms "first" and "second" are only intended for a purpose of description, and should not be understood as an indication or implication of relative importance or implicit indication of a quantity of indicated technical features. Therefore, a feature limited by "first" or "second" may explicitly or implicitly include one or more of the features. In the descriptions of embodiments of this application, unless otherwise specified, "a plurality of" means two or more than two. Orientations or location relationships indicated by terms "middle", "left", "right", "up", "down", and the like are based on orientations or location relationships shown in the accompanying drawings, and are merely intended for ease of describing this application and simplifying description, but do not indicate or imply that a specified apparatus or element needs to have a specific orientation, or be constructed and operated in a specific orientation. Therefore, the terms cannot be understood as a limitation on this application.

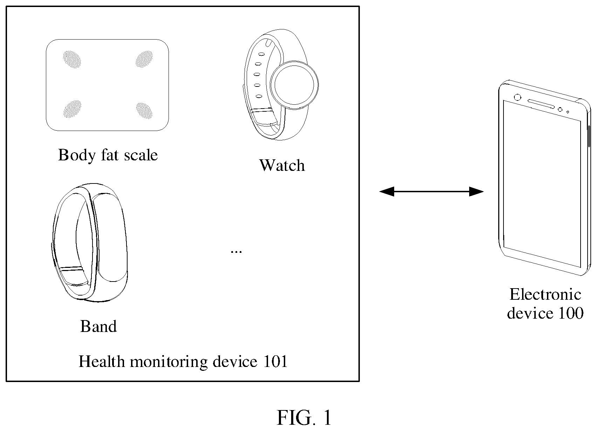

FIG. 1 is an example of a system diagram according to this application.

As shown in FIG. 1, a system may include an electronic device 100 and one or more health monitoring devices 101. The electronic device 100 and the health monitoring device 101 may be connected in a wireless communication manner. For example, a connection may be established in at least one of the following wireless connection manners: Bluetooth (Bluetooth, BT), near field communication (near field communication, NFC), wireless fidelity (wireless fidelity, Wi-Fi), or Wi-Fi Direct.

The health monitoring device 101 includes a body fat scale, a weight scale, a heart rate meter, a blood pressure meter, a smartwatch, a smart band, smart glasses, a smart ring, smart sports shoes, an electronic bracelet, an electronic necklace, an electronic accessory, an exercise apparatus (for example, a treadmill, an elliptical machine, a stepper, a fixed bicycle, or a rowing machine), a mobile phone, a computer, a tablet, or the like.

The health monitoring device 101 may detect health status information, exercise status information, emotional status information, and the like of a user through a sensor. The health status information includes a heart rate, a weight, body fat, blood pressure, blood glucose, an electroencephalogram, an electrocardiogram, an electromyogram, a body temperature, and the like. The exercise status information includes common exercise statuses such as walking, running, cycling, swimming, playing badminton, skating, surfing, and dancing, and may also include some finer-grained exercise statuses, such as forehand hit, backhand hit, Latin dancing, and popping. The emotional status information includes tension, anxiety, sadness, great pressure, excitement, joy, and the like.

The electronic device 100 may be a mobile phone, a tablet computer, a desktop computer, a laptop computer, a notebook computer, an ultra-mobile personal computer (Ultra-mobile Personal Computer, UMPC), a handheld computer, a netbook, a personal digital assistant (Personal Digital Assistant, PDA), a virtual reality device, a PDA (Personal Digital Assistant, personal digital assistant, also referred to as a palmtop computer), a portable Internet device, a data storage device, a wearable device (for example, a wireless headset, a smartwatch, a smart band, smart glasses, a head-mounted display (Head-mounted display, HMD), electronic clothing, an electronic bracelet, an electronic necklace, an electronic accessory, an electronic tattoo, and a smart mirror), a health monitoring device, an exercise apparatus (for example, a treadmill, an elliptical machine, a stepper, a fixed bicycle, and a rowing machine), or the like.

The electronic device 100 is connected to the health monitoring device 101. The health monitoring device 101 may periodically or in real time provide exercise data (including exercise target information and the foregoing exercise status information) of the user and health data (including the foregoing health status information, the emotional status information, and the like) of the user for the electronic device 100. The electronic device 100 may provide a personalized exercise recommendation for the user based on obtained exercise data of the user in different statuses (for example, a rest state, a running state, a walking state, a swimming state, or a mountain climbing state).

In embodiments of this application, exercise recommendations include a taboo-type exercise recommendation, a prescription-type exercise recommendation, and a guidance-type exercise recommendation. The taboo-type exercise recommendation includes an exercise recommendation that the user is prohibited from performing. The prescription-type exercise recommendation is related to the health data of the user, and includes an exercise recommendation that is determined based on the health data of the user and that the user is recommended to perform. The guidance-type exercise recommendation includes a universal exercise recommendation that the user is recommended to perform. When the health data of the user meets different conditions, the electronic device 100 determines different exercise recommendations.

The taboo-type exercise recommendation includes, for example, prohibiting the user from running at a speed exceeding a threshold, and for example, prohibiting a diabetes patient from exercising after taking a hypoglycemic drug, and the like. In some embodiments, the electronic device 100 may perform a corresponding operation based on the taboo-type exercise recommendation.

The prescription-type exercise recommendation includes, for example, recommending that a heart rate range of the user during an exercise is from 80 to 100 and for example, recommending that a running speed of the user is from 5 km/h to 8 km/h.

The guidance-type exercise recommendation includes, for example, recommending that the user performs a medium- or high-intensity activity for more than 30 minutes every day.

In some embodiments, the prescription-type exercise recommendation and the guidance-type exercise recommendation may be collectively referred to as a non-taboo-type exercise recommendation. The electronic device 100 may perform a corresponding operation based on the non-taboo-type exercise recommendation.

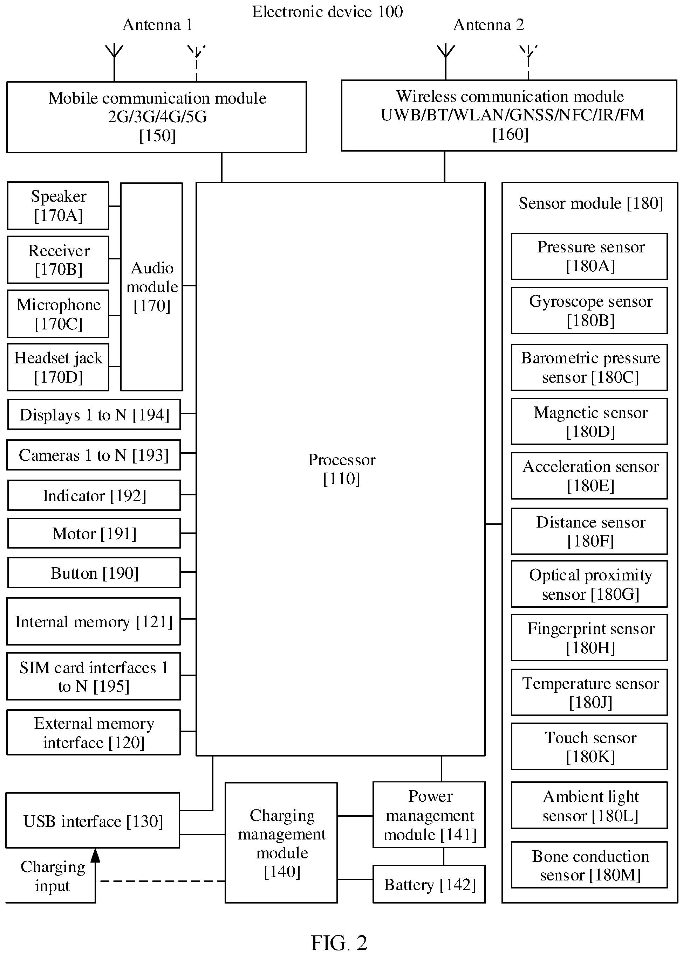

FIG. 2 is a schematic diagram of a structure of an example electronic device 100 according to an embodiment of this application.

The electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identity module (subscriber identity module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure illustrated in this embodiment of this application constitutes no specific limitation on the electronic device 100. In some other embodiments of this application, the electronic device 100 may include more or fewer components than those shown in the figure, or some components may be combined, or some components may be split, or different component arrangements may be used. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, a neural-network processing unit (neural-network processing unit, NPU), and/or the like. Different processing units may be independent devices, or may be integrated into one or more processors.

The controller may be a nerve center and a command center of the electronic device 100. The controller may generate an operation control signal based on an instruction operation code and a time sequence signal, to complete control of instruction reading and instruction execution.

A memory may further be disposed in the processor 110, and is configured to store instructions and data. In some embodiments, the memory in the processor 110 is a cache memory. The memory may store instructions or data recently used or cyclically used by the processor 110. If the processor 110 needs to use the instructions or the data again, the processor 110 may directly invoke the instructions or the data from the memory. This avoids repeated access, reduces waiting time of the processor 110, and improves system efficiency.

In some embodiments, the processor 110 may include one or more interfaces. The interfaces may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The I2C interface is a two-way synchronous serial bus, and includes a serial data line (serial data line, SDA) and a serial clock line (serial clock line, SCL). In some embodiments, the processor 110 may include a plurality of groups of I2C buses. The processor 110 may be separately coupled to the touch sensor 180K, a charger, a flash, the camera 193, and the like through different I2C bus interfaces. For example, the processor 110 may be coupled to the touch sensor 180K through the I2C interface, so that the processor 110 communicates with the touch sensor 180K through the I2C bus interface, to implement a touch function of the electronic device 100.

The I2S interface may be configured to perform audio communication. In some embodiments, the processor 110 may include a plurality of groups of I2S buses. The processor 110 may be coupled to the audio module 170 through the I2S bus, to implement communication between the processor 110 and the audio module 170. In some embodiments, the audio module 170 may transmit an audio signal to the wireless communication module 160 through the I2S interface, to implement a function of answering a call through a Bluetooth headset.

The PCM interface may also be configured to: perform audio communication, and sample, quantize, and encode an analog signal. In some embodiments, the audio module 170 may be coupled to the wireless communication module 160 through a PCM bus interface. In some embodiments, the audio module 170 may alternatively transmit the audio signal to the wireless communication module 160 through the PCM interface, to implement the function of answering a call through a Bluetooth headset. Both the I2S interface and the PCM interface may be configured to perform audio communication.

The UART interface is a universal serial data bus, and is configured to perform asynchronous communication. The bus may be a two-way communication bus. The bus converts to-be-transmitted data between serial communication and parallel communication. In some embodiments, the UART interface is usually configured to connect the processor 110 to the wireless communication module 160. For example, the processor 110 communicates with a Bluetooth module in the wireless communication module 160 through the UART interface, to implement a Bluetooth function. In some embodiments, the audio module 170 may transmit an audio signal to the wireless communication module 160 through the UART interface, to implement a function of playing music through a Bluetooth headset.

The MIPI interface may be configured to connect the processor 110 to a peripheral device such as the display 194 or the camera 193. The MIPI interface includes a camera serial interface (camera serial interface, CSI), a display serial interface (display serial interface, DSI), and the like. In some embodiments, the processor 110 communicates with the camera 193 through the CSI, to implement an image shooting function of the electronic device 100. The processor 110 communicates with the display 194 through the DSI, to implement a display function of the electronic device 100.

The GPIO interface may be configured by software. The GPIO interface may be configured to transmit a control signal or a data signal. In some embodiments, the GPIO interface may be configured to connect the processor 110 to the camera 193, the display 194, the wireless communication module 160, the audio module 170, the sensor module 180, and the like. The GPIO interface may alternatively be configured as the I2C interface, the I2S interface, the UART interface, the MIPI, or the like.

The USB interface 130 is an interface that conforms to a USB standard specification, and may specifically be a mini USB interface, a micro USB interface, a USB type-C interface, or the like. The USB interface 130 may be configured to connect to the charger to charge the electronic device 100, or may be configured to transmit data between the electronic device 100 and a peripheral device, or may be configured to connect to a headset to play audio through the headset. The interface may further be configured to connect to another electronic device such as an AR device.

It may be understood that an interface connection relationship between the modules illustrated in this embodiment of this application is merely an example for description, and does not constitute a limitation on the structure of the electronic device 100. In some other embodiments of this application, the electronic device 100 may alternatively use an interface connection manner different from that in the foregoing embodiment, or use a combination of a plurality of interface connection manners.

The charging management module 140 is configured to receive a charging input from the charger. The charger may be a wireless charger or a wired charger. In some embodiments of wired charging, the charging management module 140 may receive a charging input from the wired charger through the USB interface 130. In some embodiments of wireless charging, the charging management module 140 may receive a wireless charging input through a wireless charging coil of the electronic device 100. The charging management module 140 may further supply power to the electronic device through the power management module 141 while charging the battery 142.

The power management module 141 is configured to connect to the battery 142, the charging management module 140, and the processor 110. The power management module 141 receives an input from the battery 142 and/or the charging management module 140, and supplies power to the processor 110, the internal memory 121, an external memory, the display 194, the camera 193, the wireless communication module 160, and the like. The power management module 141 may further be configured to monitor a parameter such as a battery capacity, a battery cycle count, and a battery health status (electric leakage or impedance). In some other embodiments, the power management module 141 may alternatively be disposed in the processor 110. In some other embodiments, the power management module 141 and the charging management module 140 may alternatively be disposed in a same device.

A wireless communication function of the electronic device 100 may be implemented through the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna in the electronic device 100 may be configured to cover one or more communication frequency bands. Different antennas may further be multiplexed, to improve antenna utilization. For example, the antenna 1 may be multiplexed as a diversity antenna of a wireless local area network. In some other embodiments, the antenna may be used in combination with a tuning switch.

The mobile communication module 150 may provide a wireless communication solution that is applied to the electronic device 100 and that includes 2G, 3G, 4G, 5G, and the like. The mobile communication module 150 may include at least one filter, a switch, a power amplifier, a low noise amplifier (low noise amplifier, LNA), and the like. The mobile communication module 150 may receive an electromagnetic wave through the antenna 1, process such as filter or amplify the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1. In some embodiments, at least some functional modules of the mobile communication module 150 may be disposed in the processor 110. In some embodiments, at least some functional modules of the mobile communication module 150 may be disposed in a same device as at least some modules of the processor 110.

The modem processor may include a modulator and a demodulator. The modulator is configured to modulate a to-be-sent low-frequency baseband signal into a medium- or highfrequency signal. The demodulator is configured to demodulate a received electromagnetic wave signal into a low-frequency baseband signal. Then, the demodulator transmits the demodulated low-frequency baseband signal to the baseband processor for processing. The low-frequency baseband signal is processed by the baseband processor and then transmitted to the application processor. The application processor outputs a sound signal through an audio device (which is not limited to the speaker 170A, the receiver 170B, or the like), or displays an image or a video through the display 194. In some embodiments, the modem processor may be an independent device. In some other embodiments, the modem processor may be independent of the processor 110, and is disposed in a same device as the mobile communication module 150 or another functional module.

The wireless communication module 160 may provide a wireless communication solution that is applied to the electronic device 100 and that includes a UWB, a wireless local area network (wireless local area networks, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, and the like. The wireless communication module 160 may be one or more devices integrating at least one communication processing module. The wireless communication module 160 receives an electromagnetic wave through the antenna 2, performs frequency modulation and filtering on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In some embodiments, the antenna 1 and the mobile communication module 150 in the electronic device 100 are coupled, and the antenna 2 and the wireless communication module 160 in the electronic device 100 are coupled, so that the electronic device 100 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time division synchronous code division multiple access (time division synchronous code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a beidou navigation satellite system (beidou navigation satellite system, BDS), a quasi-zenith satellite system (quasi-zenith satellite system, QZSS), and/or a satellite-based augmentation system (satellite-based augmentation systems, SBAS).

The electronic device 100 implements a display function through the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs that execute program instructions to generate or change display information.

The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a mini-LED, a micro-LED, a micro-OLED, a quantum dot light-emitting diode (quantum dot light-emitting diodes, QLED), or the like. In some embodiments, the electronic device 100 may include one or N displays 194. N is a positive integer greater than 1.

In some embodiments of this application, the display 194 displays interface content currently output by a system. For example, the interface content is an interface provided by an instant messaging application.

The electronic device 100 may implement the image shooting function through the ISP, the camera 193, the video codec, the GPU, the display 194, the application processor, and the like.

The ISP is configured to process data fed back by the camera 193. For example, during image shooting, a shutter is pressed, and light is transmitted to a photosensitive element of the camera through a lens. An optical signal is converted into an electrical signal, and the photosensitive element of the camera transmits the electrical signal to the ISP for processing, to convert the electrical signal into a visible image. The ISP may further perform algorithm optimization on noise, brightness, and complexion of the image. The ISP may further optimize parameters such as exposure and a color temperature of an image shooting scene. In some embodiments, the ISP may be disposed in the camera 193.

The camera 193 is configured to capture a static image or a video. An optical image of an object is generated through the lens, and is projected onto the photosensitive element. The photosensitive element may be a charge coupled device (charge coupled device, CCD), or a complementary metal-oxide-semiconductor (complementary metal-oxide-semiconductor, CMOS) phototransistor. The photosensitive element converts an optical signal into an electrical signal, and then transmits the electrical signal to the ISP for converting the electrical signal into a digital image signal. The ISP outputs the digital image signal to the DSP for processing. The DSP converts the digital image signal into an image signal in a standard format such as RGB or YUV. In some embodiments, the electronic device 100 may include one or N cameras 193. N is a positive integer greater than 1.

The digital signal processor is configured to process a digital signal, and may process another digital signal in addition to the digital image signal. For example, when the electronic device 100 selects a frequency, the digital signal processor is configured to perform Fourier transform and the like on frequency energy.

The video codec is configured to compress or decompress a digital video. The electronic device 100 may support one or more types of video codecs. Therefore, the electronic device 100 may play or record videos in a plurality of coding formats, for example, moving picture experts group (moving picture experts group, MPEG)1, MPEG2, MPEG3, and MPEG4.

The NPU is a neural-network (neural-network, NN) computing processor, quickly processes input information by referring to a structure of a biological neural network, for example, by referring to a mode of transmission between human brain neurons, and may further continuously perform self-learning. Applications such as intelligent cognition of the electronic device 100 may be implemented through the NPU. Intelligent cognition includes, for example, image recognition, facial recognition, speech recognition, and text understanding.

The external memory interface 120 may be configured to connect to an external memory card, for example, a micro SD card, to extend a storage capability of the electronic device 100. The external memory card communicates with the processor 110 through the external memory interface 120, to implement a data storage function. For example, files such as music and videos are stored in the external storage card.

The internal memory 121 may be configured to store computer-executable program code. The executable program code includes instructions. The processor 110 runs the instructions stored in the internal memory 121, to perform various function applications and data processing of the electronic device 100. The internal memory 121 may include a program storage area and a data storage area. The program storage area may store an operating system, an application required by at least one function (for example, a sound playing function or an image playing function), and the like. The data storage area may store data (such as audio data and an address book) and the like that are created during use of the electronic device 100. In addition, the internal memory 121 may include a high-speed random access memory, or may include a non-volatile memory, for example, at least one magnetic disk memory, a flash memory, or a universal flash storage (universal flash storage, UFS).

The electronic device 100 may implement an audio function, for example, music playing and recording, through the audio module 170, the speaker 170A, the receiver 170B, the microphone 170C, the headset jack 170D, the application processor, and the like.

The audio module 170 is configured to convert digital audio information into an analog audio signal for output, and is also configured to convert an analog audio input into a digital audio signal. The audio module 170 may be configured to encode and decode an audio signal. In some embodiments, the audio module 170 may be disposed in the processor 110, or some functional modules of the audio module 170 are disposed in the processor 110.

The speaker 170A, also referred to as a "loudspeaker", is configured to convert an audio electrical signal into a sound signal. The electronic device 100 may be configured to listen to music or answer a hands-free call through the speaker 170A.

The receiver 170B, also referred to as an "earpiece", is configured to convert an audio electrical signal into a sound signal. When a call is answered or voice information is received through the electronic device 100, the receiver 170B may be put close to a human ear for listening to a voice.

The microphone 170C, also referred to as a "mike" or a "mic", is configured to convert a sound signal into an electrical signal. When making a call or sending a voice message, a user may make a sound near the microphone 170C through the mouth of the user, to input a sound signal to the microphone 170C. The electronic device 100 may be provided with at least one microphone 170C. In some other embodiments, the electronic device 100 may be provided with two microphones 170C, to implement a noise reduction function, in addition to collecting a sound signal. In some other embodiments, the electronic device 100 may alternatively be provided with three, four, or more microphones 170C, to collect a sound signal, implement noise reduction, identify a sound source, implement a directional recording function, and the like.

The headset jack 170D is configured to connect to a wired headset. The headset jack 170D may be a USB interface 130, or may be a 3.5 mm open mobile terminal platform (open mobile terminal platform, OMTP) standard interface or cellular telecommunications industry association of the USA (cellular telecommunications industry association of the USA, CTIA) standard interface.

The pressure sensor 180A is configured to sense a pressure signal, and may convert the pressure signal into an electrical signal. In some embodiments, the pressure sensor 180A may be disposed in the display 194. In some optional embodiments of this application, the pressure sensor 180A may be configured to: capture a pressure value generated when a finger part of the user touches the display, and transmit the pressure value to the processor, so that the processor identifies a finger part through which the user enters a user operation.

There are a plurality of types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes is changed. The electronic device 100 determines pressure intensity based on a capacitance change. When a touch operation is performed on the display 194, the electronic device 100 detects intensity of the touch operation through the pressure sensor 180A. The electronic device 100 may further calculate a touch position based on a detection signal of the pressure sensor 180A. In some embodiments, different touch positions may correspond to different operation instructions. In some optional embodiments, the pressure sensor 180A may further calculate a quantity of touch points based on a detected signal, and transmit a calculated value to the processor, so that the processor identifies that the user enters a user operation through a single finger or a plurality of fingers.

The gyroscope sensor 180B may be configured to determine an exercise posture of the electronic device 100. In some embodiments, an angular velocity of the electronic device 100 around three axes (axes X, Y, and Z of the electronic device) may be determined through the gyroscope sensor 180B. The gyroscope sensor 180B may be configured to implement image stabilization during image shooting. For example, when the shutter is pressed, the gyroscope sensor 180B detects a shaking angle of the electronic device 100, and calculates, based on the angle, a distance for which a lens module needs to compensate, so that shaking of the electronic device 100 is canceled through reverse motion of the lens, thereby implementing image stabilization. The gyroscope sensor 180B may further be used in a navigation scenario and a somatic game scenario.

The barometric pressure sensor 180C is configured to measure barometric pressure. In some embodiments, the electronic device 100 calculates an altitude by using the barometric pressure measured by using the barometric pressure sensor 180C, to assist in positioning and navigation.

The magnetic sensor 180D includes a Hall sensor. The electronic device 100 may detect opening and closing of a flip cover through the magnetic sensor 180D. In some embodiments, when the electronic device 100 is a clamshell phone, the electronic device 100 may detect opening and closing of a flip cover through the magnetic sensor 180D. Further, a feature such as automatic unlocking of the flip cover is set based on a detected opening or closing state of the leather case or a detected opening or closing state of the flip cover.

The acceleration sensor 180E may detect accelerations in various directions (usually on three axes) of the electronic device 100. When the electronic device 100 is still, a magnitude and a direction of gravity may be detected. The acceleration sensor 180E may further be configured to identify a posture of the electronic device, and is used in an application such as switching between a landscape mode and a portrait mode or a pedometer. In some optional embodiments of this application, the acceleration sensor 180E may be configured to capture an acceleration value generated when a finger part of the user touches the display (or a finger of the user taps a rear side bezel of a rear housing of the electronic device 100), and transmit the acceleration value to the processor, so that the processor identifies a finger part through which the user enters a user operation.

The distance sensor 180F is configured to measure a distance. The electronic device 100 may measure the distance in an infrared manner or a laser manner. In some embodiments, the electronic device 100 may measure a distance in an image shooting scene through the distance sensor 180F, to implement quick focusing.

The optical proximity sensor 180G may include, for example, a light-emitting diode (LED) and an optical detector, for example, a photodiode. The light-emitting diode may be an infrared light-emitting diode. The electronic device 100 emits infrared light by using the light-emitting diode. The electronic device 100 detects infrared reflected light from a nearby object by using the photodiode. When sufficient reflected light is detected, the electronic device 100 may determine that there is an object near the electronic device 100. When insufficient reflected light is detected, the electronic device 100 may determine that there is no object near the electronic device 100. The electronic device 100 may detect, through the optical proximity sensor 180G, that the user holds the electronic device 100 close to an ear for a call, to automatically turn off the display for power saving. The optical proximity sensor 180G may also be used for automatic screen unlocking and locking in a smart cover mode or a pocket mode.

The ambient light sensor 180L is configured to sense ambient light brightness. The electronic device 100 may adaptively adjust brightness of the display 194 based on the sensed ambient light brightness. The ambient light sensor 180L may further be configured to automatically adjust white balance during image shooting. The ambient light sensor 180L may further cooperate with the optical proximity sensor 180G to detect whether the electronic device 100 is in a pocket, to avoid an accidental touch.

The fingerprint sensor 180H is configured to collect a fingerprint. The electronic device 100 may use a feature of the collected fingerprint to implement fingerprint-based unlocking, application lock access, fingerprint-based image shooting, fingerprint-based call answering, and the like.

The temperature sensor 180J is configured to detect a temperature. In some embodiments, the electronic device 100 implements a temperature processing policy based on a temperature detected by the temperature sensor 180J. For example, when a temperature reported by the temperature sensor 180J exceeds a threshold, the electronic device 100 lowers performance of a processor near the temperature sensor 180J, to reduce power consumption for thermal protection. In some other embodiments, when the temperature is lower than another threshold, the electronic device 100 heats the battery 142 to avoid abnormal shutdown of the electronic device 100 caused by a low temperature. In some other embodiments, when the temperature is lower than still another threshold, the electronic device 100 boosts an output voltage of the battery 142 to avoid abnormal shutdown caused by a low temperature.

The touch sensor 180K is also referred to as a "touch panel". The touch sensor 180K may be disposed in the display 194, and the touch sensor 180K and the display 194 constitute a touchscreen, which is also referred to as a "touch screen". The touch sensor 180K is configured to detect a touch operation performed on or near the touch sensor 180K. The touch operation is an operation that the user touches the display 194 by using a hand, an elbow, a stylus, or the like. The touch sensor may transfer the detected touch operation to the application processor to determine a type of a touch event. A visual output related to the touch operation may be provided on the display 194. In some other embodiments, the touch sensor 180K may alternatively be disposed on a surface of the electronic device 100 at a position different from that of the display 194.

The bone conduction sensor 180M may obtain a vibration signal. In some embodiments, the bone conduction sensor 180M may obtain a vibration signal of a vibration bone of a human vocal-cord part. The bone conduction sensor 180M may also be in contact with a human pulse, to receive a blood pressure beating signal. In some embodiments, the bone conduction sensor 180M may alternatively be disposed in the headset, to combine into a bone conduction headset. The audio module 170 may obtain a voice signal through parsing based on the vibration signal that is of the vibration bone of the vocal-cord part and that is obtained by the bone conduction sensor 180M, to implement a voice function. The application processor may parse heart rate information based on the blood pressure beating signal obtained by the bone conduction sensor 180M, to implement a heart rate detection function.

The button 190 includes a power button, a volume button, and the like. The button 190 may be a mechanical button, or may be a touch button. The electronic device 100 may receive a button input, and generate a button signal input related to a user setting and function control of the electronic device 100.

The motor 191 may generate a vibration prompt. The motor 191 may be configured to provide an incoming call vibration prompt or a touch vibration feedback. For example, touch operations performed on different applications (for example, image shooting and audio playback) may correspond to different vibration feedback effects. The motor 191 may also correspond to different vibration feedback effects for touch operations performed on different areas of the display 194. Different application scenarios (for example, a time reminder, information receiving, an alarm clock, and a game) may also correspond to different vibration feedback effects. A touch vibration feedback effect may further be customized.

The indicator 192 may be an indicator light, and may be configured to indicate a charging status and a power change, or may be configured to indicate a message, a missed call, a notification, and the like.

The SIM card interface 195 is configured to connect to a SIM card. The SIM card may be inserted into the SIM card interface 195 or withdrawn from the SIM card interface 195, to implement contact with or separation from the electronic device 100.

It may be understood that the structure illustrated in this application constitutes no specific limitation on the electronic device 100. In some other embodiments, the electronic device 100 may include more or fewer components than those shown in the figure, or some components may be combined, or some components may be split, or different component arrangements may be used. The components shown in the figure may be implemented by hardware, software, or a combination of software and hardware.

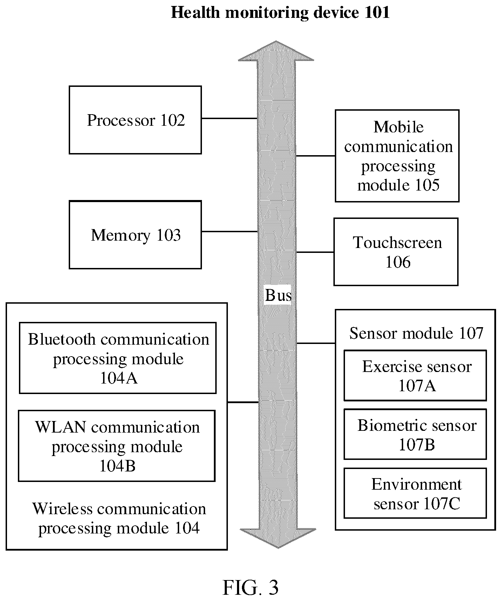

FIG. 3 is an example of a schematic diagram of a structure of a health monitoring device 101 according to this application.

As shown in FIG. 3, the health monitoring device 101 may include a processor 102, a memory 103, a wireless communication processing module 104, a mobile communication processing module 105, a touchscreen 106, and a sensor module 107. These components may be connected through a bus.

The processor 102 may be configured to read and execute computer-readable instructions. In a specific implementation, the processor 102 may mainly include a controller, an arithmetic unit, and a register. The controller is mainly responsible for decoding instructions and sending control signals for operations corresponding to the instructions. The arithmetic unit is mainly responsible for performing a fixed-point or floating-point arithmetic operation, a shift operation, a logic operation, and the like, and may also perform an address operation and address translation. The register is mainly responsible for storing a quantity of register operations, intermediate operation results, and the like that are temporarily stored during instruction execution. In a specific implementation, a hardware architecture of the processor 102 may be an application-specific integrated circuit (Application Specific Integrated Circuits, ASIC) architecture, an MIPS architecture, an ARM architecture, an NP architecture, or the like.

In some embodiments, the processor 102 may be configured to parse signals received by the wireless communication processing module 104 and the mobile communication processing module 105, for example, a request message that is sent by an electronic device 100 and that is for obtaining exercise data of a user, or a request message that is sent by the electronic device 100 and that is for stopping obtaining the exercise data of the user. The processor 102 may be configured to perform corresponding analysis and processing based on information collected by the sensor module 107, for example, analyze a health status, an exercise status, and an emotional status of the user.

The memory 103 is coupled to the processor 102, and is configured to store at least one of various software programs or a plurality of groups of instructions. In a specific implementation, the memory 103 may include a high-speed random access memory, and may also include a non-volatile memory, for example, one or more magnetic disk storage devices, flash devices, or other non-volatile solid-state storage devices. The memory 103 may store an operating system, for example, an embedded operating system such as uCOS, VxWorks, or RTLinux. The memory 103 may further store a communication program. The communication program may be used for communicating with the electronic device 100, one or more servers, or an additional device.

The wireless communication processing module 104 may include one or more of a Bluetooth (BT) communication processing module 104A and a WLAN communication processing module 104B.

In some embodiments, the one or more of the Bluetooth (BT) communication processing module and the WLAN communication processing module may listen to a signal, for example, a detection request or a scan signal transmitted by another device (for example, the electronic device 100), and may send a response signal, for example, a detection response or a scan response, so that the another device (for example, the electronic device 100) can discover the health monitoring device 101. The health monitoring device 101 establishes a wireless communication connection to the another device (for example, the electronic device 100), and communicates with the another device (for example, the electronic device 100) by using one or more wireless communication technologies such as Bluetooth or a WLAN.

In some other embodiments, the one or more of the Bluetooth (BT) communication processing module and the WLAN communication processing module may also transmit a signal, for example, a broadcast Bluetooth signal or a beacon signal, so that the another device (for example, the electronic device 100) may discover the health monitoring device 101. The health monitoring device establishes a wireless communication connection to the another device (for example, the electronic device 100) and communicates with the another device (for example, the electronic device 100) by using the wireless communication technologies such as Bluetooth or a WLAN.

The mobile communication processing module 105 may provide a wireless communication solution that is applied to the electronic device 100 and that includes 2G, 3G, 4G, 5G, and the like. The mobile communication processing module 105 is configured to perform cellular communication and/or data communication. For example, the mobile communication processing module 105 may include a circuit switching module (a "CS" module) for performing cellular communication and a packet switching module (a "PS" module) for performing data communication. In this application, the mobile communication processing module 105 may communicate with another device (for example, a server) by using a fourth generation mobile communication technology (4th generation mobile networks) or a fifth generation mobile communication technology (5th generation mobile networks).

The touchscreen 106 (touch panel) is also referred to as a touch panel, and is an inductive liquid crystal display apparatus that may receive an input signal such as a touch, and may be configured to display an image, a video, and the like. The touchscreen 106 may be a liquid crystal display (liquid crystal display, LCD), an organic light-emitting diode (organic light-emitting diode, OLED) display, an active-matrix organic light-emitting diode (active-matrix organic light-emitting diode, AMOLED) display, a flexible light-emitting diode (flexible light-emitting diode, FLED) display, a quantum dot light-emitting diode (quantum dot light-emitting diodes, QLED) display, or the like.

The sensor module 107 may include an exercise sensor 107A, a biometric sensor 107B, an environment sensor 107C, and the like.

The exercise sensor 107A is an element that converts a non-electricity change (such as a speed or pressure) into an electricity change. The exercise sensor 107A may include at least one of the following: an acceleration sensor, a gyroscope sensor, a geomagnetic sensor (also referred to as an electronic compass sensor), an atmospheric pressure sensor, or the like. The acceleration sensor may detect a magnitude of acceleration in each direction (generally three axes, namely, an x axis, a y axis, and a z axis). The gyroscope sensor may be configured to determine an exercise posture. The electronic compass sensor may be configured to measure a direction, implementing or assisting navigation. The atmospheric pressure sensor is configured to measure atmospheric pressure. In some embodiments, a height change of a location may be calculated by using a weak atmospheric pressure change in an exercise process. In addition, precision may be controlled within 10 cm in an exercise process at a height of a 10-story building, and data from rock climbing to stairs climbing may be detected.

In this application, the exercise sensor 107A may measure an activity status of the user, for example, a quantity of running steps, a speed, a quantity of swimming circles, a cycling distance, and an exercise posture (for example, playing a ball, swimming, or running).

The biometric sensor 107B is an instrument that is sensitive to biogenic substance and converts a concentration of the biogenic substance into an electrical signal for detection. The biometric sensor 107B is an analysis tool or system including immobilized biosensitive materials (including bioactive substances, such as enzymes, antibodies, antigens, microorganisms, cells, tissue, and nucleic acids) as identification elements and appropriate physicochemical transducers (such as oxygen electrodes, photodiodes, field effect transistors, and piezoelectric crystals), that is, signal amplification apparatuses. The biometric sensor has functions of a receiver and a converter. The biometric sensor 107B may include at least one of the following: a blood glucose sensor, a blood pressure sensor, an electrocardiogram sensor, an electromyogram sensor, a body temperature sensor, an electroencephalogram sensor, and the like. Functions mainly implemented by these sensors include health and medical monitoring, entertainment, and the like.

The blood glucose sensor is configured to measure blood glucose. The blood pressure sensor is configured to measure blood pressure. The electrocardiogram sensor, for example, uses silver nanowires to monitor an electrophysiological signal, such as an electrocardiogram. The electromyogram sensor is configured to monitor an electromyogram. The body temperature sensor is configured to measure a body temperature, and the electroencephalogram sensor is configured to monitor a brain wave. In this application, the biometric sensor 107B may measure physiological indexes (such as blood glucose, blood pressure, a body temperature, and an electrocardiogram) of the user, and the health monitoring device 101 may deduce a health status of the user based on the physiological indexes.

The biometric sensor 107B may further include a heart rate sensor and a galvanic skin response sensor.

The heart rate sensor may track exercise intensity, different exercise training modes, and the like of the user by detecting a heart rate of the user, and may deduce health data such as a sleep period and sleep quality of the user. When capacitive light is emitted to skin, light reflected by skin tissue is received by a photosensitive sensor and converted into an electrical signal. Then, the electrical signal is converted into a digital signal. The heart rate can be calculated based on an absorption rate of blood.

The galvanic skin response sensor is configured to measure a degree of awakening the user, and the degree of awakening is closely related to a degree of attention and a degree of participation of the user, and is usually disposed on a device that may monitor a sweat level. Skin resistance and conductance of a human body vary with functionality of skin sweat glands. These measurable skin electrical changes may be referred to as skin electrical activities (EDA).

In this embodiment of this application, the health monitoring device 101 measures a psychologically caused sweat gland activity by using the galvanic skin response sensor, to determine a psychological activity of the user, for example, a mood index of the user. For example, the mood is happy, nervous and afraid, great pressure, or the like.

The environment sensor 107C may include at least one of the following: an air temperature and humidity sensor, a rain volume sensor, a light sensor, a wind speed and direction sensor, a particulate matter sensor, and the like. The environment sensor 107C may detect air quality, for example, detect a haze degree, an indoor formaldehyde concentration, and PM2.5. In this application, the environment sensor 107C may measure a weather change, air humidity, air quality, and the like.

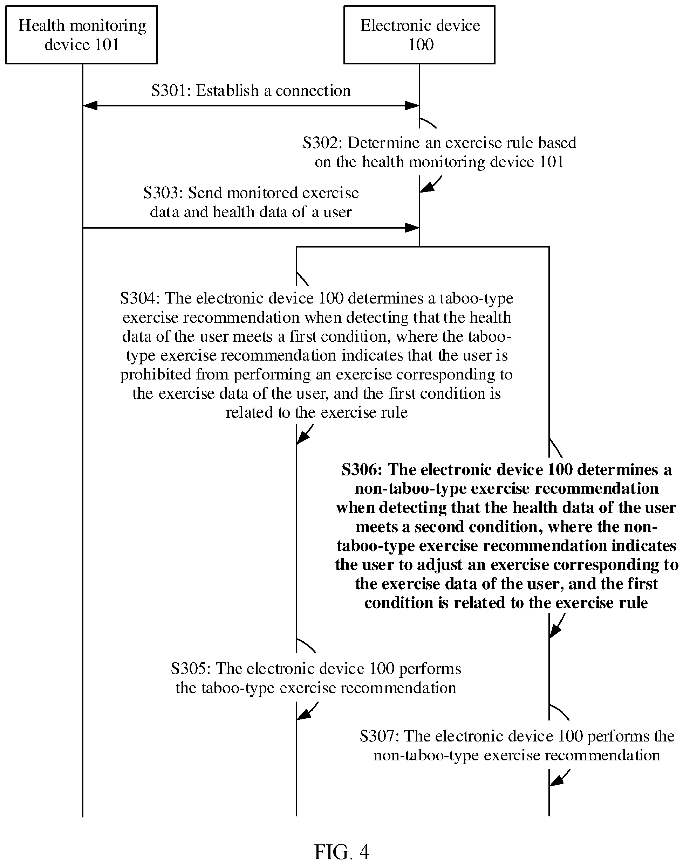

It may be understood that a structure shown in FIG. 4 does not constitute a specific limitation on the health monitoring device 101. Optionally, in some other embodiments of this application, the wearable device 101 may include more or fewer components than those shown in the figure, or some components may be combined, or some components may be split, or different component arrangements may be used. The components shown in the figure may be implemented by hardware, software, or a combination of software and hardware.

In this embodiment of this application, the electronic device 100 establishes a connection to one or more health monitoring devices 101, and the electronic device 100 may obtain the exercise data and the health data of the user based on the health monitoring devices 101. The exercise data and the health data of the user may include sensor data detected by the health monitoring device 101 through at least one sensor. In this application, the sensor data includes but is not limited to data detected by at least one of the exercise sensor 107A, the biometric sensor 107B, and the environment sensor 107C.

Optionally, the exercise data and the health data of the user may be sent to the electronic device 100 after the health monitoring device 101 performs analysis and processing based on the sensor data. Optionally, the exercise data and the health data of the user may be obtained after the electronic device 100 obtains the sensor data sent by the health monitoring device 101 and then performs analysis and processing based on the sensor data.

The electronic device 100 may determine an exercise recommendation for the user based on the obtained exercise data and health data of the user.

In some embodiments, the health data of the user includes data detected by at least one sensor and prestored personal data of the user. The personal data of the user includes information, such as an age, a gender, a height, a medical history, and an allergic history of the user. The personal data of the user may be provided by one or more health monitoring devices 101, or may be prestored in the electronic device 100. When the exercise data and personal data of the user meet different conditions, the electronic device 100 determines different exercise recommendations (a taboo-type exercise recommendation, a prescription-type exercise recommendation, and a guidance-type exercise recommendation).

In some specific scenarios, the electronic device 100 is a smartphone, and the electronic device 100 establishes a connection to one or more health monitoring devices 101. When the health monitoring devices 101 detect that the user is running, the electronic device 100 receives health data that the user is running provided by the health monitoring devices 101. The electronic device 100 may output an exercise recommendation for the user in real time based on the health data that the user is running and with reference to the personal data of the user. The exercise recommendation may be: For example, it is detected that you are running and your heart rate is high, it is recommended that you slow down a running speed to less than 5 km/h. For another example, it is detected that you are running and running duration exceeds two hours, it is recommended that you stop exercising. For another example, it is detected that you are running and you are a heart disease patient, you are prohibited from continuing to run. Please stop running.

In some other specific scenarios, the electronic device 100 is a treadmill, and the electronic device 100 establishes a connection to one or more health monitoring devices 101. When the treadmill detects that the treadmill is started, the treadmill may output an exercise recommendation for the user in real time with reference to the personal data of the user. For example, it is detected that you are on a treadmill and you are a heart disease patient, you are prohibited from running. Please stop running. Alternatively, when the health monitoring devices 101 detect that the user is running and the treadmill receives the health data that the user is running provided by the health monitoring devices 101, the treadmill may output the foregoing exercise recommendation for the user in real time based on the health data that the user is running and with reference to the personal data of the user. Optionally, the electronic device 100 may perform a corresponding operation based on the exercise recommendation. For example, in a current scenario, the treadmill (the electronic device 100) may be stopped.

The following describes a step procedure of an exercise guidance method provided in this application. As shown in FIG. 4, the method may include the following steps.

Step S301: An electronic device 100 establishes a connection to a health monitoring device 101.

The electronic device 100 and the health monitoring device 101 may be connected to a local area network in a wired or wireless fidelity (wireless fidelity, Wi-Fi) connection manner, or may communicate with each other through a mobile network or the Internet, or may establish a communication connection in a near field communication (Near field communication, NFC) manner, to implement data communication.

For example, the electronic device 100 and the health monitoring device 101 may be in a same local area network, and a Wi-Fi link is established between the electronic device 100 and the health monitoring device 101 by using a Wi-Fi protocol, to implement communication between the devices. Specifically, the electronic device 100 and the health monitoring device 101 establish a peer to peer (peer to peer, P2P) connection, or access a same router.

Optionally, a Bluetooth link may alternatively be established between the electronic device 100 and the health monitoring device 101 by using a Bluetooth protocol, to implement communication between the devices based on the Bluetooth link; or the electronic device 100 and the health monitoring device 101 may be interconnected through a cellular network; or the electronic device 100 and the health monitoring device 101 may be interconnected through a transfer device (for example, a USB data cable or a dock device), to implement communication.

In some embodiments, when the electronic device 100 and the health monitoring device 101 are connected to a local area network of a same AP, the electronic device 100 and the health monitoring device 101 are neighboring devices or adjacent devices of each other. The electronic device 100 may discover one or more other online devices in the same local area network, and the one or more other devices include the health monitoring device 101.

In some embodiments, when the electronic device 100 communicates with the health monitoring device 101 through a mobile network or the Internet, the electronic device 100 may discover, through the mobile network or the Internet, one or more other devices whose login accounts are an account of the electronic device 100 or one or more other devices whose login accounts are an associated account of the login account of the electronic device 100, and the one or more other devices include the health monitoring device 101. Devices that log in to a same account or an associated account may obtain device information of each other to implement data communication. The associated account may be an account authorized by a same account.

The account may be an account provided by a cloud server provider for a user, for example, a Huawei account, or may be an account used to log in to an application, for example, an account of various types of communication software or an account of payment software.

Step S302: The electronic device 100 determines an exercise rule based on the health monitoring device 101.

After the electronic device 100 establishes the connection to the health monitoring device 101, the electronic device 100 determines the exercise rule based on the health monitoring device 101. The exercise rule includes a taboo-type exercise rule and a non-taboo-type exercise rule that are related to data detected by the health monitoring device, and different health monitoring devices 101 correspond to different exercise rules.

In some embodiments, the non-taboo-type exercise rule includes a prescription-type exercise rule and a guidance-type exercise rule.

The taboo-type exercise rule includes an exercise recommendation for an exercise that the user is prohibited from performing. The prescription-type exercise rule is related to personal data of the user, and includes an exercise recommendation that is determined based on the personal data of the user and that the user is recommended to perform. The guidance-type exercise rule is related to a device type of the health monitoring device 101, and includes a universal exercise recommendation that the user is recommended to perform.

For example, when the health monitoring device 101 is a heart rate meter, an exercise rule of the heart rate meter may include a taboo-type exercise rule and a non-taboo-type exercise rule.

Taboo-type exercise rule: In a non-exercise state, if a heart rate exceeds 130 times/minute or is less than 50 times/minute, the user is prohibited from performing a high-intensity exercise (for example, fast running, mountain climbing, or swimming). In an exercise state, if the heart rate exceeds 160 times/minute, the user is prohibited from continuing to exercise, and so on.

Non-taboo-type exercise rule: In the exercise state, an appropriate exercise heart rate is (170 - an age of the user) times/minute. For example, for a 50-year-old person, an appropriate exercise heart rate is about 120 times/minute. Keeping an appropriate exercise heart rate for more than 30 minutes every day is helpful for physical health, and so on.

For example, when the health monitoring device 101 is a thermometer, an exercise rule of the thermometer may include a taboo-type exercise rule.

Taboo-type exercise rule: When a body temperature of the user exceeds 38°C, the user is prohibited from performing a high-intensity exercise (for example, fast running, mountain climbing, or swimming).

For example, when the health monitoring device 101 is a smart band, a function of the smart band includes measuring a heart rate and measuring a body temperature. In this case, an exercise rule of the smart band may include related exercise rules of the heart rate meter and the thermometer.

For example, when the health monitoring device 101 is a blood pressure monitor, an exercise rule of the blood pressure monitor may include a taboo-type exercise rule and a non-taboo-type exercise rule.

Taboo-type exercise rule: Users with low blood pressure (for example, systolic pressure lower than 90 millimeters of mercury and/or diastolic pressure lower than 60 millimeters of mercury) and users with high blood pressure (for example, systolic pressure higher than 140 millimeters of mercury and/or diastolic pressure higher than 90 millimeters of mercury) are prohibited from performing a high-intensity exercise (for example, fast running, mountain climbing, or swimming). The users with high blood pressure are prohibited from performing an anaerobic exercise (for example, weightlifting or a barbell exercise), and so on.

Non-taboo-type exercise rule: It is recommended that the users with high blood pressure perform a low-intensity aerobic exercise (for example, jogging, rope jumping, mountain climbing, swimming, or cycling).

For example, when the health monitoring device 101 is a blood glucose meter, an exercise rule of the blood glucose meter may include a taboo-type exercise rule and a non-taboo-type exercise rule.

Taboo-type exercise rule: Users with a low blood glucose level (for example, adults with a blood glucose concentration lower than 3.92 mmol/L, and infants or children with a blood glucose concentration lower than 2.22 mmol/L) are prohibited from performing a high-intensity exercise. Users with a high blood glucose level (for example, a blood glucose concentration higher than 7 mmol/L) are prohibited from performing a high-intensity anaerobic exercise, and so on.

Non-taboo-type exercise rule: It is recommended that the users with a high blood glucose level (for example, adults with a blood glucose concentration higher than 7 mmol/L) perform an appropriate aerobic exercise. For the users with a high blood glucose level, keeping exercising for more than 30 minutes every day helps control blood glucose, and so on.

In some embodiments, if the health monitoring device 101 and the electronic device 100 are connected for the first time, the health monitoring device 101 provides an exercise rule of the health monitoring device 101 for the electronic device 100, and the electronic device 100 may store an identity and the exercise rule of the health monitoring device 101. When the health monitoring device 101 and the electronic device 100 are connected next time, the electronic device 100 may determine the exercise rule of the health monitoring device 101 based on the identity of the health monitoring device 101.

In some embodiments, the connection is established between the health monitoring device 101 and the electronic device 100, the electronic device 100 sends an instruction to the health monitoring device 101 to obtain an exercise rule corresponding to the health monitoring device 101, and the health monitoring device 101 sends the corresponding exercise rule to the electronic device 100 based on the instruction.

In some embodiments, the electronic device 100 determines an exercise rule based on the device type of the health monitoring device 101. For example, the electronic device 100 detects that the health monitoring device 101 is the heart rate meter. In this case, the electronic device 100 determines the exercise rule of the heart rate meter. For example, the electronic device 100 detects that the health monitoring device 101 is the smart band, and the function of the smart band includes measuring the heart rate and measuring the body temperature. In this case, the electronic device 100 determines the exercise rule of the smart band, including the related exercise rules of the heart rate meter and the thermometer.

In this embodiment of this application, the electronic device 100 may establish a connection to one or more health monitoring devices 101, and then determine an exercise rule of the connected health monitoring device 101. The exercise rule determined by the electronic device 100 based on the health monitoring device 101 may be provided by the health monitoring device 101, or may be stored in the electronic device 100.

Step S303: The health monitoring device 101 sends detected exercise data and health data of the user to the electronic device 100.

The health monitoring device 101 continuously or periodically sends the detected exercise data and health data of the user to the electronic device 100. The exercise data of the user includes exercise status information, exercise target information, and the like. The exercise status information is a detected real-time exercise status, and includes, for example, walking, running, cycling, swimming, playing badminton, skating, surfing, and dancing. The exercise target information is preset exercise data, and includes, for example, a target speed parameter, a target duration parameter, a target distance parameter, and a target calorie parameter that are set by the user before exercising. The health data of the user includes health status information (such as a heart rate, a weight, body fat, blood pressure, blood glucose, an electroencephalogram, an electrocardiogram, an electromyogram, and a body temperature), emotional status information (such as tension, anxiety, sadness, great pressure, excitement, and joy), and the like.

In some embodiments, the health monitoring device 101 sends the detected exercise data and health data of the user to the electronic device 100 based on a received instruction. The instruction may be triggered by the user, or may be sent by the electronic device 100 to the health monitoring device 101, or may be sent by another device to the health monitoring device 101.

Optionally, before the health monitoring device 101 sends the detected exercise data and health data of the user to the electronic device 100, if a time difference between detection time of latest data (the exercise data and the health data of the user) stored by the health monitoring device 101 and current time exceeds a threshold, the health monitoring device 101 re-detects and obtains current exercise data and health data, and the health monitoring device 101 stores the redetected exercise data and health data as latest data, and sends the latest data to the electronic device 100. When the time difference between the detection time of the latest data stored by the health monitoring device and the current time does not exceed the threshold, the health monitoring device 101 sends the latest data stored by the health monitoring device to the electronic device 100.

In some embodiments, the health monitoring device 101 detects the exercise data and the health data of the user based on the received instruction, and sends the detected exercise data and health data of the user to the electronic device 100.

In some embodiments, when detecting that the exercise data of the user meets a preset condition, the health monitoring device 101 continuously or periodically sends the detected exercise data of the user to the electronic device 100. For example, the health monitoring device 101 is the heart rate meter, and the heart rate meter detects that the heart rate of the user increases greatly. It may be determined that the user is in a non-quiet state (for example, an exercise state or an emotional excitement state). In this case, the heart rate meter may increase a periodic frequency or continuously send detected heart rate data of the user to the electronic device 100.

In some embodiments, the exercise data and the health data of the user obtained by the electronic device 100 include time information. The time information may be time when the health monitoring device 101 detects the exercise data and the health data of the user, or may be time when the health monitoring device 101 sends the exercise data and the health data of the user to the electronic device 100, or may be time when the electronic device 100 obtains the exercise data and the health data of the user. The electronic device 100 may determine, based on the time information, whether to re-obtain the exercise data and the health data of the user from the health monitoring device 101.

Optionally, after obtaining the exercise data and the health data of the user, the electronic device 100 stores the exercise data and the health data of the user. Before the electronic device 100 determines an exercise recommendation based on the exercise data and the health data of the user, the electronic device 100 determines, based on the time information of the exercise data and the health data of the user, whether a time difference between the time information and current time exceeds a threshold. If the duration exceeds the threshold, the electronic device 100 re-obtains the exercise data and the health data of the user from the health monitoring device 101.

Optionally, the electronic device 100 outputs prompt information to prompt the user to re-measure the health data of the user through the health monitoring device 101.

In some embodiments, the exercise data and the health data of the user may be provided by a plurality of health monitoring devices 101. For example, the health monitoring device 101 includes a heart rate meter, a blood glucose meter, a smart band, a treadmill, and the like. The heart rate meter provides heart rate data for the electronic device 100, the blood glucose meter provides blood glucose data for the electronic device 100, and the smart band and the treadmill provide the exercise data of the user for the electronic device 100 (including exercise data such as a speed and time, or an exercise status such as running, walking, or mountain climbing).

Optionally, the health monitoring device 101 may provide the exercise data of the user for the electronic device 100, and the electronic device 100 detects the health data of the user based on a sensor of the electronic device 100. For example, the health monitoring device 101 includes a treadmill, and the treadmill provides the exercise data of the user for the electronic device 100. The electronic device 100 includes a mobile phone, a smartwatch, a smart band, a blood glucose meter, and the like, and the electronic device 100 detects the health data of the user based on the sensor of the electronic device 100.

Optionally, the health monitoring device 101 may provide the health data of the user for the electronic device 100, and the electronic device 100 detects the exercise data of the user based on the sensor of the electronic device 100. For example, the health monitoring device 101 includes a heart rate meter, a blood glucose meter, a smart band, and the like, and the electronic device 100 includes a mobile phone, a smartwatch, a smart band, an exercise apparatus, and the like.

In some embodiments, the health monitoring device 101 and the electronic device 100 are a same electronic device.

Steps S301 to S303 are optional. The electronic device 100 may detect the exercise data and the health data of the user based on the sensor of the electronic device 100, and an exercise rule is determined by the electronic device 100 based on a sensor function of the electronic device 100.

Step S304: The electronic device 100 determines a taboo-type exercise recommendation when detecting that the health data of the user meets a first condition, where the taboo-type exercise recommendation indicates that the user is prohibited from performing an exercise corresponding to the exercise data of the user.

After obtaining the exercise data and the health data of the user, the electronic device 100 detects that the health data of the user meets the first condition, and the electronic device 100 determines the taboo-type exercise recommendation. The taboo-type exercise recommendation includes an exercise recommendation that the user is prohibited from performing, and indicates that the user is prohibited from performing the exercise corresponding to the exercise data of the user. The first condition is related to the exercise rule determined based on the health monitoring device 101.

In some embodiments, the first condition is related to a taboo-type exercise rule in the exercise rule determined based on the health monitoring device 101.

In some embodiments, the first condition may be that the health data of the user exceeds a threshold. For example, the health monitoring device 101 is a heart rate meter. In this case, the health data of the user includes heart rate data. After establishing a connection to the heart rate meter, the electronic device 100 determines an exercise rule that corresponds to the heart rate meter and that includes a taboo-type exercise rule and a non-taboo-type exercise rule. The taboo-type exercise rule includes, for example, that a heart rate exceeds 160 times/minute, so that the user is prohibited from exercising. In this case, the first condition may specifically be that the heart rate data exceeds 160 times/minute. The heart rate meter sends detected heart rate data to the electronic device 100. When the heart rate data exceeds 160 times/minute, the electronic device 100 outputs a taboo-type exercise recommendation. That is, the user is prohibited from exercising.

Optionally, the electronic device 100 determines the taboo-type exercise rule corresponding to the heart rate meter. For example, the taboo-type exercise rule includes: When a heart rate exceeds 130 times/minute or is less than 50 times/minute in a non-exercise state, the user is prohibited from performing a high-intensity exercise (for example, fast running, mountain climbing, or swimming); and when the heart rate exceeds 160 times/minute in an exercise state, the user is prohibited from continuing to exercise. In this case, the first condition may be that the exercise data of the user exceeds a threshold in the exercise state. For example, the first condition may specifically be that the heart rate data exceeds 130 times/minute in the non-exercise state, or the heart rate data exceeds 160 times/minute in the exercise state. The heart rate meter sends the detected heart rate data to the electronic device 100. When the heart rate data exceeds 130 times/minute, the electronic device 100 determines whether the user is in the exercise state. Optionally, the electronic device 100 determines, based on data obtained by an exercise sensor (an acceleration sensor, a gyroscope sensor, a geomagnetic sensor, or an atmospheric pressure sensor), whether the user is in the exercise state. If the user is not in the exercise state, the electronic device 100 outputs the taboo-type exercise recommendation. That is, the user is prohibited from exercising. If the user is in the exercise state, the electronic device does not output the taboo-type exercise recommendation, and the user keeps exercising. When the heart rate data exceeds 160 times/minute, the electronic device 100 determines that the user is currently in the exercise state, and the electronic device 100 outputs a taboo-type exercise recommendation. That is, the user is prohibited from continuing to exercise.

For another example, the health monitoring device 101 is a heart rate meter and a thermometer, so that the health data of the user includes the heart rate data and body temperature data. After establishing the connection to the health monitoring device 101, the electronic device 100 determines taboo-type exercise rules corresponding to the heart rate meter and the thermometer. For example, the taboo-type exercise rules include: A heart rate exceeds 160 times/minute, so that the user is prohibited from exercising. A body temperature exceeds 38°C, so that the user is prohibited from exercising. In this case, the first condition may specifically be that the heart rate exceeds 160 times/minute or the body temperature exceeds 38°C.

In some embodiments, the health data of the user may include both the health data detected in real time by the electronic device 100 or the health monitoring device 101 and prestored information such as an age, a gender, a height, a medical history, and an allergic history of the user. The health data of the user may be provided by the health monitoring device 101, or may be prestored in the electronic device 100. With reference to the health data of the user, the electronic device 100 may provide a taboo-type exercise recommendation for the user. Optionally, the first condition is related to the health data of the user.

For example, the health monitoring device 101 is a blood glucose meter, so that the health data of the user includes blood glucose data. After establishing a connection to the blood glucose meter, the electronic device 100 determines a taboo-type exercise rule corresponding to the blood glucose meter. For example, the taboo-type exercise rule includes: When a diabetes patient takes a hypoglycemic drug, a blood glucose level is physiologically reduced, so that the user is prohibited from exercising within a period of time. When the electronic device 100 detects, through the health monitoring device 101, that a blood glucose level of the user suddenly decreases, and the medical history in the health data of the user records that the user suffers from diabetes, if the user is currently in the exercise state, the electronic device 100 outputs a taboo-type exercise recommendation. That is, the user is prohibited from continuing to exercise.

In some embodiments, the exercise data of the user may include both the exercise data detected in real time by the electronic device 100 or the health monitoring device 101 and planned exercise data of the user. The planned exercise data of the user includes an exercise plan, an exercise target, and the like. The electronic device 100 determines, based on the planned exercise data of the user and the health data of the user, that the health data of the user meets the first condition, and outputs a taboo-type exercise recommendation. That is, the user is prohibited from performing an exercise corresponding to the planned exercise data.

For example, when the user stands on a treadmill, and the treadmill obtains an exercise parameter (the planned exercise data), for example, a speed of 8 km/h that is set by the user, before the user starts running, the electronic device 100 (the treadmill or a device that establishes a connection to the treadmill) outputs, based on the planned exercise data and with reference to an obtained current heart rate of the user of more than 130 times/minute (where the health data of the user meets the first condition), a taboo-type exercise recommendation, indicating that the user cannot run at a speed of 8 km/h.

Optionally, the electronic device 100 receives the planned exercise data of the user, determines, with reference to prestored personal data of the user (for example, including information such as an age, a gender, a height, a medical history, and an allergic history of the user), that the personal data of the user meets the first condition, and outputs a taboo-type exercise recommendation. That is, the user is prohibited from performing the exercise corresponding to the planned exercise data.

For example, when the user stands on a treadmill and sets an exercise parameter (the planned exercise data), for example, a speed of 5 km/h on the treadmill, and the treadmill obtains the exercise parameter that is set by the user (running at a speed of 5 km/h), before the user starts running, the electronic device 100 (the treadmill or a device that establishes a connection to the treadmill) may output, based on the planned exercise data and with reference to an obtained medical history of the user that records that the user suffers from a heart disease, a taboo-type exercise recommendation: The user suffering from a heart disease is prohibited from running.

For another example, the medical history of the user records that the user suffers from diabetes, and the electronic device 100 detects, through the health monitoring device 101, that a blood glucose level of the user suddenly decreases. When the user stands on a treadmill and the treadmill obtains an exercise parameter that is set by the user, before the user starts running, the electronic device 100 (the treadmill or a device that establishes a connection to the treadmill) may output, based on the health data of the user (where the user suffers from diabetes and it is detected that the blood glucose level suddenly decreases) and with reference to obtained planned exercise data (running on the treadmill), a taboo-type exercise recommendation: The user suffering from diabetes is prohibited from running.

In some embodiments, the health data of the user obtained by the electronic device 100 includes time information. If a time difference between the time information of the health data of the user and current time exceeds a threshold, the electronic device 100 outputs a taboo-type exercise recommendation. That is, the user is prohibited from exercising, and the user needs to re-detect the health data of the user.

For example, the health data of the user includes blood glucose, and blood glucose data obtained by the electronic device 100 is blood glucose data at 10:00 a.m. When the user stands on a treadmill at 5:00 p.m. and intends to run, the treadmill obtains an exercise parameter that is set by the user. The blood glucose data obtained by the electronic device 100 (the treadmill or a device that establishes a connection to the treadmill) is the blood glucose data at 10:00 a.m., and an interval between 10:00 a.m. and 5:00 p.m. exceeds the threshold. Therefore, before the user starts running, the electronic device 100 outputs, based on the blood glucose data, a taboo-type exercise recommendation. That is, the user is prohibited from running, and the user needs to re-detect the blood glucose.

Optionally, the electronic device 100 outputs, with reference to the prestored personal data of the user, a taboo-type exercise recommendation if the duration between the time information of the health data of the user and the current time exceeds the threshold. For example, the medical history of the user records that the user suffers from diabetes, blood glucose data obtained by the electronic device 100 is blood glucose data at 10 a.m., and an interval between 10 a.m. and 5:00 p.m. exceeds the threshold. In this case, the electronic device 100 outputs, based on the blood glucose data, a taboo-type exercise recommendation. That is, the user is prohibited from running, and the user needs to re-detect the blood glucose.

In some embodiments, the electronic device 100 determines, based on the obtained exercise data and health data of the user and with reference to environment data, that the health data of the user meets the first condition, and outputs a taboo-type exercise recommendation. The environment data includes information such as a haze degree, an indoor formaldehyde concentration, PM2.5 detection, a weather change, air humidity, and air quality. The environment data may be provided by the health monitoring device 101, or may be detected by the electronic device 100. With reference to the environment data, the electronic device 100 may provide a taboo-type exercise recommendation for the user. For example, if a weather condition at current time is haze, visibility is within 20 meters, and the user is currently in an outdoor exercise state, the electronic device 100 outputs a taboo-type exercise recommendation. That is, the user is prohibited from continuing to exercise.

In some embodiments, the taboo-type exercise recommendation includes an exercise recommendation that the user is prohibited from performing, and further includes an exercise recommendation that may be performed. For example, a patient with high blood pressure may perform an appropriate aerobic exercise (for example, running or cycling) instead of an anaerobic exercise (for example, weightlifting or a barbell exercise). When the user uses a weightlifting apparatus, the electronic device 100 detects that the user is lifting a weight (the exercise data of the user). With reference to the obtained medical history of the user that records that the user suffers from high blood pressure, the electronic device 100 may output a taboo-type exercise recommendation: The user suffering from high blood pressure is prohibited from lifting a weight. It is recommended that the user run on a treadmill. It can be learned that the taboo-type exercise recommendation includes an exercise recommendation that the user is prohibited from performing, that is, the user suffering from high blood pressure is prohibited from lifting a weight; and the taboo-type exercise recommendation further includes an exercise recommendation that may be performed, that is, it is recommended that the user run on a treadmill.

For another example, if a weather condition at current time is haze, visibility is within 20 meters, and the user is currently in an outdoor exercise state, the electronic device 100 outputs a taboo-type exercise recommendation. That is, the user is prohibiting from continuing to exercise outdoors. The taboo-type exercise recommendation may further include an exercise recommendation that may be performed. For example, it is recommended that the user exercise indoors.

Optionally, the electronic device 100 may further provide indication information for an exercise recommendation that may be performed in the taboo-type exercise recommendation. The indication information may indicate the user to perform the exercise recommendation and may include, for example, execution location information, direction information, apparatus information, an exercise manner, exercise site information, and an exercise apparatus store link. For example, the electronic device 100 outputs a taboo-type exercise recommendation: The user suffering from high blood pressure is prohibited from lifting a weight. It is recommended that the user run on a treadmill. The taboo-type exercise recommendation may further include a location of the treadmill, to indicate the user to quickly find the treadmill. For another example, the electronic device 100 outputs a taboo-type exercise recommendation: The user is prohibited from continuing to exercise outdoors. It is recommended that the user exercise indoors. The taboo-type exercise recommendation may further include a location of an indoor gym or an exercise site near the user, and may further include a route to the location of the indoor gym or the exercise site near the user, and the like.

In some embodiments, after receiving a user instruction, the electronic device 100 detects that the health data of the user meets the first condition, and determines a taboo-type exercise recommendation.

For example, when the electronic device receives a user instruction for starting exercising before the user exercises, the electronic device 100 determines, based on the planned exercise data and the health data of the user, a taboo-type exercise recommendation (where for example, you are prohibited from exercising by using the planned exercise data based on your health data).

For another example, when the electronic device receives a user instruction for obtaining an exercise recommendation in an exercise process of the user, the electronic device 100 determines a taboo-type exercise recommendation based on the exercise data and the health data of the user.

For another example, after the user ends an exercise, when the electronic device receives a user instruction indicating that the exercise ends, the electronic device 100 determines a taboo-type exercise recommendation based on the exercise data and the health data of the user in the exercise time period.

In some embodiments, after determining a taboo-type exercise recommendation, the electronic device 100 outputs the taboo-type exercise recommendation.

Optionally, after the electronic device 100 determines the taboo-type exercise recommendation, the electronic device 100 may indicate the health monitoring device 101 to output the taboo-type exercise recommendation. If the electronic device 100 is connected to a plurality of health monitoring devices 101, the electronic device 100 may indicate any one or more of the health monitoring devices 101 to output the taboo-type exercise recommendation.

Optionally, the electronic device 100 may indicate another electronic device to output the taboo-type exercise recommendation, and the another electronic device is an electronic device that may implement data communication with the electronic device 100. For example, if the electronic device 100 is connected to a wearable device (for example, a watch, a band, or a headset), the electronic device 100 determines, based on the exercise data of the user and an exercise rule, that the exercise data of the user meets the first condition, and the electronic device 100 indicates the wearable device to output the taboo-type exercise recommendation. An output manner includes but is not limited to a pop-up window, text, a voice, a video, a projection device, or the like.

In some embodiments, the electronic device 100 outputs the taboo-type exercise recommendation, and may output the taboo-type exercise recommendation on another electronic device simultaneously. The another electronic device may be an electronic device that establishes an association relationship with the electronic device 100. For example, the electronic device 100 may output the taboo-type exercise recommendation to a mobile phone of a relative of a user of the electronic device 100, to prompt the relative of the user to prevent the user of the electronic device 100 from exercising. Optionally, the electronic device 100 may send the taboo-type exercise recommendation to another electronic device in a form of a notification, an SMS message, or the like.

In some embodiments, the electronic device 100 receives a user instruction after determining the taboo-type exercise recommendation. In response to the user instruction, the electronic device 100 outputs the taboo-type exercise recommendation.

For example, when the user stands on a treadmill, and the treadmill obtains exercise data, for example, a speed of 5 km/h that is set by the user, before the user starts running, if the treadmill receives a user instruction for starting running (for example, a user operation for a running start button), in response to the user instruction, the electronic device 100 (the treadmill or a device that establishes a connection to the treadmill) outputs, based on the exercise data and with reference to an obtained current heart rate of the user of more than 160 times/minute (where the health data of the user meets the first condition), a taboo-type exercise recommendation: For example, the user is prohibited from running at a speed of 5 km/h.

Optionally, step S305: The electronic device 100 performs the taboo-type exercise recommendation.

After determining the taboo-type exercise recommendation, the electronic device 100 performs the taboo-type exercise recommendation. For example, when the electronic device 100 is an exercise apparatus, if the taboo-type exercise recommendation is that the user is prohibited from exercising, the electronic device 100 performs the taboo-type exercise recommendation. That is, the electronic device 100 (the exercise apparatus) is stopped. Specifically, when the electronic device 100 is a treadmill, if the taboo-type exercise recommendation is that the user is prohibited from running or exercising, the treadmill performs the taboo-type exercise recommendation. That is, the treadmill is stopped.

In some embodiments, after performing the taboo-type exercise recommendation, the electronic device 100 outputs the taboo-type exercise recommendation. For example, when the user runs on a treadmill, and the taboo-type exercise recommendation determined by the electronic device 100 (the treadmill or a device that establishes a connection to the treadmill) is that the user is prohibited from running or exercising, the treadmill performs the taboo-type exercise recommendation. That is, the treadmill is stopped. After the treadmill is stopped, the electronic device 100 outputs the taboo-type exercise recommendation.

Optionally, after performing the taboo-type exercise recommendation, the electronic device 100 outputs the taboo-type exercise recommendation. In addition, the electronic device 100 may provide a withdrawal control for withdrawing execution of the taboo-type exercise recommendation. When the electronic device 100 detects a user operation for the withdrawal control, the electronic device 100 withdraws execution of the taboo-type exercise recommendation. For example, in the foregoing example of stopping the treadmill, after the treadmill is stopped, the electronic device 100 outputs the taboo-type exercise recommendation. In addition, the electronic device 100 provides the withdrawal control. When the electronic device 100 detects the user operation for the withdrawal control, starting of the treadmill is resumed.

In some embodiments, after outputting the taboo-type exercise recommendation, the electronic device 100 receives a user instruction for the taboo-type exercise recommendation. In response to the user instruction, the electronic device 100 performs the taboo-type exercise recommendation. Optionally, the taboo-type exercise recommendation includes an execution control, and the user triggers the execution control. In this case, the electronic device 100 performs the taboo-type exercise recommendation in response to the trigger instruction.

In some embodiments, the electronic device 100 indicates another electronic device to perform the taboo-type exercise recommendation. The another electronic device is an electronic device that may implement data communication with the electronic device 100. For example, the electronic device 100 establishes a connection to a treadmill. When the user stands on the treadmill and sets an exercise parameter (the planned exercise data), for example, a speed of 5 km/h on the treadmill, and the treadmill obtains the exercise parameter that is set by the user (running at a speed of 5 km/h), before the user starts running, the electronic device 100 may output, based on the planned exercise data and with reference to an obtained medical history of the user that records that the user suffers from a heart disease, a taboo-type exercise recommendation: The user suffering from a heart disease is prohibited from running. In addition, the electronic device 100 may synchronously send an instruction to the treadmill, to indicate the treadmill to perform the taboo-type exercise recommendation. That is, the treadmill is stopped.

In this embodiment of this application, the electronic device 100 establishes a connection to one or more health monitoring devices 101, and the electronic device 100 may obtain the exercise data and the health data of the user based on the health monitoring devices 101. The electronic device 100 determines a taboo-type exercise recommendation for the user based on the obtained exercise data and health data of the user. The taboo-type exercise recommendation provides an exercise recommendation that the user is prohibited from performing, to help the user avoid an exercise that cannot be performed by the user. The following step S306 is a manner of determining a non-taboo-type exercise recommendation by the electronic device 100. The taboo-type exercise recommendation provides an exercise recommendation that may be performed by the user. Step S306 and step S304 are parallel steps, and there is no sequence.

Step S306: The electronic device 100 determines a non-taboo-type exercise recommendation when detecting that the health data of the user meets a second condition, where the non-taboo-type exercise recommendation indicates the user to adjust an exercise corresponding to the exercise data of the user.

After obtaining the exercise data and the health data of the user, the electronic device 100 detects that the health data of the user meets the second condition, and the electronic device 100 determines the non-taboo-type exercise recommendation. The non-taboo-type exercise recommendation includes a prescription-type exercise recommendation and a guidance-type exercise recommendation. The prescription-type exercise recommendation is related to the personal data of the user, and includes an exercise recommendation that is determined based on the personal data of the user and that the user is recommended to perform. The guidance-type exercise recommendation is related to the device type of the health monitoring device 101, and includes a universal exercise recommendation that the user is recommended to perform. The non-taboo-type exercise recommendation indicates the user to adjust the exercise corresponding to the exercise data of the user. The second condition is related to the exercise rule determined based on the health monitoring device 101.

In some embodiments, the second condition is related to the non-taboo-type exercise rule in the determined exercise rule of the health monitoring device 101. For example, the health monitoring device 101 is a blood glucose meter. In this case, the exercise data of the user includes blood glucose data. After establishing a connection to the blood glucose meter, the electronic device 100 determines a non-taboo-type exercise rule of the blood glucose meter. For example, the non-taboo-type exercise rule includes: Daily medium- and high-intensity exercise duration of at least 30 minutes helps control blood glucose. When the electronic device 100 detects that the user stops exercising after exercising for 20 minutes, the electronic device 100 outputs a non-taboo-type exercise recommendation. That is, it is recommended that the user extend the exercise duration to more than 30 minutes.

In some embodiments, the second condition may be that the health data of the user exceeds a threshold. For example, the health monitoring device 101 is a heart rate meter. In this case, the health data of the user includes heart rate data. After establishing a connection to the heart rate meter, the electronic device 100 determines an exercise rule that corresponds to the heart rate meter and that includes a taboo-type exercise rule and a non-taboo-type exercise rule. For example, the non-taboo-type exercise rule includes: In an exercise state, an appropriate exercise heart rate is (170 - an age of the user) times/minute. For example, for a 50-year-old person, an appropriate exercise heart rate is about 120 times/minute. In this case, the second condition may specifically be that the heart rate data exceeds 130 times/minute. The heart rate meter sends detected heart rate data to the electronic device 100. When the heart rate data exceeds 130 times/minute, the electronic device 100 outputs a non-taboo-type exercise recommendation. That is, it is recommended that the user reduce exercise intensity.

For example, the user stands on a treadmill and runs at a speed of 8 km/h on the treadmill, the health monitoring device 101 is a heart rate meter, and the heart rate meter continuously sends heart rate data to the electronic device 100. When the heart rate data exceeds 130 times/minute, the electronic device 100 outputs, based on a current exercise status (running at a speed of 8 km/h) of the user, a non-taboo-type exercise recommendation: It is recommended that the user reduce exercise intensity and run at a speed of 4 km/h.

In some embodiments, the health data of the user may include both the health data detected in real time by the electronic device 100 or the health monitoring device 101 and prestored information such as an age, a gender, a height, a medical history, and an allergic history of the user. The health data of the user may be provided by the health monitoring device 101, or may be prestored in the electronic device 100. With reference to the health data of the user, the electronic device 100 may provide a non-taboo-type exercise recommendation for the user. Optionally, the second condition is related to the health data of the user.

In some embodiments, the exercise data of the user may include both the exercise data detected in real time by the electronic device 100 or the health monitoring device 101 and planned exercise data of the user. The planned exercise data of the user includes an exercise plan, an exercise target, and the like. The electronic device 100 determines, based on the planned exercise data of the user and the health data of the user, that the exercise data of the user meets the second condition, and outputs a non-taboo-type exercise recommendation.

For example, when the user stands on a treadmill, and the treadmill obtains an exercise parameter (the planned exercise data), for example, a speed of 8 km/h that is set by the user, before the user starts running, the electronic device 100 (the treadmill or a device that establishes a connection to the treadmill) outputs, based on the planned exercise data and with reference to an obtained current heart rate of the user of more than 130 times/minute (where the health data of the user meets the second condition), a non-taboo-type exercise recommendation: It is recommended that the user run at a speed of 4 km/h at the current time.

Optionally, the electronic device 100 receives the planned exercise data of the user, determines, with reference to prestored health data of the user (including information such as an age, a gender, a height, a medical history, and an allergic history of the user), that the health data of the user meets the second condition, and outputs a non-taboo-type exercise recommendation. That is, an exercise corresponding to the planned exercise data is adjusted.

In some embodiments, the planned exercise data of the user is an exercise target, the electronic device 100 determines the exercise target, and the electronic device 100 provides a non-taboo-type exercise recommendation based on the exercise target and the exercise data of the user. With an exercise target of weight reduction as an example, the following shows an example of a manner of generating a non-taboo-type exercise recommendation. The non-taboo-type exercise recommendation indicates an exercise status that can efficiently implement the exercise target.

After the electronic device 100 collects weight data for n times in n different time periods, the electronic device 100 invokes exercise data stored in the electronic device 100 in the time period, and calculates an exercise manner that may enable the user to efficiently achieve the exercise target of weight reduction. The following describes the calculation method.

It is known that a measured weight value of the user is $Y = \{y_1, y_2 \dots y_n\}$, and it is known that in a time period of weight measurement of the user, an exercise status of the user includes $S = \{s_1, s_2 \dots s_m\}$. The exercise status includes a rest state, a running state, a walking state, a swimming state, a mountain climbing state, a dancing state, or the like. The exercise status may further include a fine-grained exercise status, for example, a state of running at 5 km/h, a state of running at 8 km/h, Latin dancing, popping, or the like.

$$T = \begin{bmatrix} t_{1,1} & \cdots & t_{1,n} \\ \vdots & \ddots & \vdots \\ t_{n,1} & \cdots & t_{n,m} \end{bmatrix}$$

It is known that maintenance duration is $\qquad$ in weight measurement duration and in the exercise status of $\{s_1, s_2 \dots s_m\}$. Similar to the maintenance duration, it is known that a heart rate of the user is

$$H = \begin{bmatrix} h_{1,1} & \cdots & h_{1,n} \\ \vdots & \ddots & \vdots \\ h_{n,1} & \cdots & h_{n,m} \end{bmatrix}$$

in the weight measurement duration and in the exercise status of $\{s_1, s_2 \dots s_n\}$, and a coefficient corresponding to the exercise status $\{s_1, s_2 \dots s_m\}$ is set to $X = \{x_1, x_2 \dots x_m\}$.

Linear equations are solved as follows:

$$\begin{cases} t_{1,1}h_{1,1}x_{1,1} + t_{1,2}h_{1,2}x_{1,2} + \cdots + t_{1,m}h_{1,m}x_{1,m} = y_1 \\ \dots \\ t_{n,1}h_{n,1}x_{n,1} + t_{n,2}h_{n,2}x_{n,2} + \cdots + t_{n,m}h_{n,m}x_{n,m} = y_n \end{cases}$$

The coefficient $X = \{x_1, x_2 \dots x_m\}$ corresponding to each exercise status is calculated. An exercise status with a maximum coefficient is an exercise status in which the user efficiently achieves the exercise target. The electronic device 100 outputs a non-taboo-type exercise recommendation. The non-taboo-type exercise recommendation indicates that the exercise status with the maximum coefficient is the exercise status in which the user efficiently achieves the exercise target. The exercise target is weight reduction. In this case, $\{y_1, y_2 \ldots y_n\}$ is weight data. The foregoing uses the heart rate as a calculation parameter for weight reduction. Consumed calories, blood pressure, or the like may also be used as a calculation parameter for exercise target implementation. This is not limited in this application.

Optionally, the exercise target may alternatively be body fat reduction. In this case, $\{y_1, y_2 \ldots y_n\}$ is body fat data. The exercise target may alternatively be increasing maximum oxygen uptake. In this case, $\{y_1, y_2 \ldots y_n\}$ is maximum oxygen uptake data, or the like.

In some embodiments, the electronic device 100 determines, based on the obtained exercise data and health data of the user and with reference to the environment data, that the exercise data of the user meets the second condition, and outputs a non-taboo-type exercise recommendation: For example, it is recommended that the user exercise indoors due to a weather reason.

In some embodiments, for a non-taboo-type exercise recommendation, the electronic device 100 may further provide indication information for indicating the user to perform the exercise recommendation. For example, the indication information may include execution location information, direction information, apparatus information, an exercise manner, exercise site information, an exercise apparatus store link, or the like. For example, the electronic device 100 outputs a non-taboo-type exercise recommendation: It is recommended that the user run on a treadmill. The non-taboo-type exercise recommendation may further include a location of the treadmill, to indicate the user to quickly find the treadmill. For another example, the electronic device 100 outputs a non-taboo-type exercise recommendation: It is recommended that the user exercises indoors. The non-taboo-type exercise recommendation may further include a location of an indoor gym or an exercise site near the user, and may further include a route to the location of the indoor gym or the exercise site near the user, and the like.

In some embodiments, after receiving a user instruction, the electronic device 100 detects that the health data of the user meets the second condition, and determines a non-taboo-type exercise recommendation.

For example, when the electronic device receives a user instruction for starting exercising before the user exercises, the electronic device 100 determines, based on the planned exercise data and the health data of the user, a non-taboo-type exercise recommendation (for example, based on your health data, it is recommended that you reduce exercise intensity of the planned exercise data).

For another example, when the electronic device receives a user instruction for obtaining an exercise recommendation in an exercise process of the user, the electronic device 100 determines a non-taboo-type exercise recommendation based on the exercise data and the health data of the user.

For another example, after the user ends an exercise, when the electronic device receives a user instruction indicating that the exercise ends, the electronic device 100 determines a non-taboo-type exercise recommendation based on the exercise data and the health data of the user in the exercise time period.

In some embodiments, after determining a non-taboo-type exercise recommendation, the electronic device 100 outputs the non-taboo-type exercise recommendation.

Optionally, after the electronic device 100 determines the non-taboo-type exercise recommendation, the electronic device 100 may indicate the health monitoring device 101 to output the non-taboo-type exercise recommendation. If the electronic device 100 is connected to a plurality of health monitoring devices 101, the electronic device 100 may indicate any one or more of the health monitoring devices 101 to output the non-taboo-type exercise recommendation.

Optionally, the electronic device 100 may indicate another electronic device to output the non-taboo-type exercise recommendation, and the another electronic device includes an electronic device that may implement data communication with the electronic device 100. Optionally, if the electronic device 100 is connected to a wearable device (for example, a watch, a band, or a headset), the electronic device 100 determines, based on the exercise data of the user and an exercise rule, that the exercise data of the user meets the first condition, and the electronic device 100 indicates the wearable device to output the non-taboo-type exercise recommendation. An output manner includes but is not limited to a pop-up window, text, a voice, a video, projection, or the like. Optionally, the electronic device 100 may send the non-taboo-type exercise recommendation to another electronic device in a form of a notification, an SMS message, or the like.

In some embodiments, after determining the non-taboo-type exercise recommendation, the electronic device 100 receives a user instruction. In response to the user instruction, the electronic device 100 outputs the non-taboo-type exercise recommendation.

For example, when the user stands on a treadmill, and the treadmill obtains exercise data, for example, a speed of 8 km/h that is set by the user, before the user starts running, if the treadmill receives a user instruction (for example, a user operation for a running start button), in response to the user instruction, the electronic device 100 (the treadmill or a device that establishes a connection to the treadmill) outputs, based on the exercise data and with reference to an obtained current heart rate of the user of more than 130 times/minute (where the health data of the user meets the second condition), a non-taboo-type exercise recommendation: For example, it is recommended that exercise intensity be reduced, and a speed be adjusted to 4 km/h for running.

Optionally, step S307: The electronic device 100 performs the non-taboo-type exercise recommendation.

After determining the non-taboo-type exercise recommendation, the electronic device 100 performs the non-taboo-type exercise recommendation.

For example, when the electronic device 100 is a treadmill, if the non-taboo-type exercise recommendation is: It is recommended that the user reduce the exercise intensity and run at a speed of 4 km/h, the treadmill performs the non-taboo-type exercise recommendation, and adjusts the exercise parameter of the treadmill to a speed of 4 km/h.

In some embodiments, after determining the non-taboo-type exercise recommendation, the electronic device 100 outputs the non-taboo-type exercise recommendation, and performs the non-taboo-type exercise recommendation.

In some embodiments, after performing the non-taboo-type exercise recommendation, the electronic device 100 outputs the non-taboo-type exercise recommendation. For example, when the electronic device 100 is a treadmill, if the non-taboo-type exercise recommendation is: It is recommended that the user reduce running intensity and adjust a running speed to 4 km/h, the treadmill performs the non-taboo-type exercise recommendation. That is, the treadmill adjusts the running speed to 4 km/h. After the treadmill adjusts the running speed to 4 km/h, the non-taboo-type exercise recommendation is displayed on a screen of the treadmill.

In some embodiments, after outputting the non-taboo-type exercise recommendation, the electronic device 100 receives a user instruction for the non-taboo-type exercise recommendation. In response to the user instruction, the electronic device 100 performs the non-taboo-type exercise recommendation. Optionally, the non-taboo-type exercise recommendation includes an execution control, and the user triggers the execution control. In this case, the electronic device 100 performs the non-taboo-type exercise recommendation in response to the trigger instruction.

For example, the electronic device 100 is a treadmill, and the treadmill outputs a non-taboo-type exercise recommendation. That is, it is recommended that the user reduce running intensity and adjust a running speed to 4 km/h. In addition, the non-taboo-type exercise recommendation prompts the user whether the running speed needs to be adjusted to 4 km/h. When the user chooses to adjust the running speed to 4 km/h, in response to the user operation, the treadmill performs the non-taboo-type exercise recommendation and automatically adjusts the running speed to 4 km/h.

For another example, before the user runs, the user sets the planned exercise data to exercising for 20 minutes. The electronic device outputs a non-taboo-type exercise recommendation based on the planned exercise data and the health data of the user. That is, it is recommended that the user extend the exercise duration to more than 30 minutes. In addition, the non-taboo-type exercise recommendation prompts the user whether running duration needs to be adjusted to 30 minutes. When the user chooses to adjust the running duration to 30 minutes, in response to the user operation, the treadmill performs the non-taboo-type exercise recommendation and automatically adjusts the running duration to 30 minutes.

In some embodiments, the electronic device 100 indicates another electronic device to perform the non-taboo-type exercise recommendation. The another electronic device includes an electronic device that may implement data communication with the electronic device 100. For example, the electronic device 100 establishes a connection to a treadmill. When the user stands on the treadmill and sets an exercise parameter (the planned exercise data), for example, a speed of 8 km/h on the treadmill, the user starts to run at the speed of 8 km/h. When the health data of the user meets the second condition, for example, a heart rate of the user exceeds 130 times/minute, the electronic device 100 may output a non-taboo-type exercise recommendation: It is recommended that the user reduce exercise intensity and run at a speed of 4 km/h. In addition, the electronic device 100 may synchronously send an instruction to the treadmill, to indicate the treadmill to perform the non-taboo-type exercise recommendation. That is, the exercise parameter of the treadmill is automatically adjusted to the speed of 4 km/h.

In this embodiment of this application, the electronic device 100 establishes a connection to one or more health monitoring devices 101, and the electronic device 100 may obtain the exercise data and the health data of the user based on the health monitoring devices 101. The electronic device 100 determines a taboo-type exercise recommendation or a non-taboo-type exercise recommendation for the user based on the obtained exercise data and health data of the user. The taboo-type exercise recommendation provides an exercise recommendation that the user is prohibited from performing, and the non-taboo-type exercise recommendation provides an exercise recommendation that the user is recommended to perform. According to this embodiment of this application, an exercise that cannot be performed by the user can be avoided, and an exercise that can be performed by the user is recommended, to provide a personalized exercise solution for the user. The electronic device 100 may further perform a corresponding operation based on a determined exercise recommendation, to implement automatic execution of the exercise recommendation.

An exercise guidance method provided in this embodiment of this application may include more or fewer steps than step S301 to step S307, or some steps may be combined, or some steps may be split, or some steps may be added, or a different step implementation may be used.

In some embodiments, the electronic device 100 continuously or periodically receives the exercise data of the user sent by the health monitoring device 101. The electronic device 100 determines received exercise data of the user and the health data of the user. The health data of the user meets the second condition. In this case, the electronic device 100 outputs a non-taboo-type exercise recommendation. Then, the electronic device 100 determines exercise data of the user received next time. The health data of the user meets the first condition. In this case, the electronic device 100 outputs a taboo-type exercise recommendation. Optionally, the electronic device 100 determines the exercise data of the user received next time and the health data of the user. The health data of the user meets the second condition. In this case, the electronic device 100 outputs a non-taboo-type exercise recommendation. It can be learned that the electronic device 100 may continuously determine changed health data of the user based on the exercise data of the user continuously or periodically received and the health data of the user, to output a corresponding exercise recommendation.

In some embodiments, the electronic device 100 continuously or periodically receives the exercise data and the health data of the user sent by the health monitoring device 101. The electronic device 100 outputs the exercise recommendation only when the exercise recommendation determined by the electronic device 100 is changed. If exercise recommendations determined by the electronic device 100 are the same, the same exercise recommendation is not repeatedly output. In some embodiments, the electronic device 100 does not output a same exercise recommendation within preset duration. After the preset duration is exceeded, the electronic device 100 may output the determined exercise recommendation.

Based on the foregoing embodiments, the following describes two specific application scenarios applicable to the exercise guidance method in embodiments of this application by using examples.

Application scenario 1: The user exercises on an indoor treadmill.

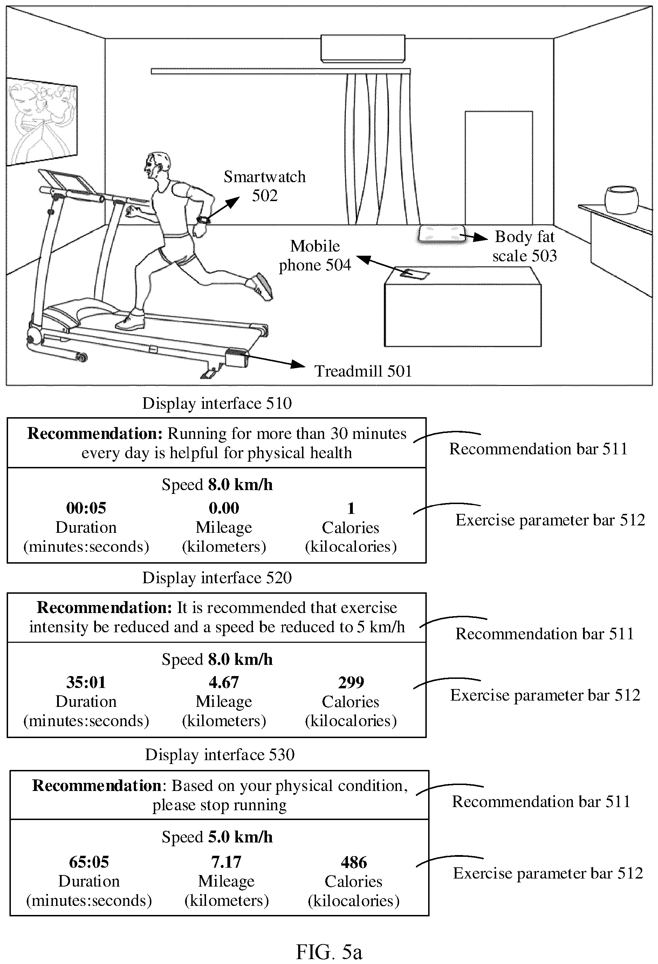

As shown in FIG. 5a, the electronic device 100 may be a treadmill 501, and the health monitoring device 101 may include electronic devices such as a smartwatch 502, a body fat scale 503, and a mobile phone 504. The smartwatch 502 may provide information such as a heart rate and a body temperature of the user, the body fat scale 503 may provide information such as a weight and body fat of the user, and the mobile phone 504 may provide prestored health data of the user (including information such as an age, a gender, a height, a medical history, and an allergic history of the user).

When the user stands on the treadmill 501 to exercise, and the user sets an exercise parameter (for example, a speed), that is, planned exercise data of the user on the treadmill 501, the treadmill 501 receives the exercise data. With reference to information provided by the smartwatch 502, the body fat scale 503, or the mobile phone 504, the treadmill 501 determines an exercise recommendation for the user and outputs the exercise recommendation. For example, a display interface 510 shown in FIG. 5a is displayed on a display of the treadmill 501. The display interface 510 includes a recommendation bar 511 and an exercise parameter bar 512. The recommendation bar 511 is used to output an exercise recommendation that is for the user and that is determined by the treadmill 501, and the exercise parameter bar 512 is used to output current exercise data of the user, for example, including a speed, duration, a mileage, and calories. It can be learned that the current user just starts to exercise for 5 seconds, and the treadmill 501 determines that the health data of the user meets the second condition (for example, a heart rate is within a normal range and there is no special medical history), and displays a determined exercise recommendation (non-taboo-type exercise recommendation) for the user in the recommendation bar 511: Running for more than 30 minutes every day is helpful for physical health.

The electronic device 100 may continuously or periodically receive the exercise data and the health data of the user provided by one or more health monitoring devices 101. The electronic device 100 may output an exercise recommendation for the user in real time based on the exercise data of the user and with reference to the health data of the user. After the user exercises on the treadmill 501 for 35 minutes, the treadmill 501 determines that the health data of the user meets a third condition (for example, a heart rate exceeds a first threshold), and a display interface 520 shown in FIG. 5a is displayed on the display of the treadmill 501. The display interface 520 displays, in a recommendation bar 511, a determined exercise recommendation (non-taboo-type exercise recommendation) for the user: It is recommended that exercise intensity be reduced, and a speed be reduced to 5 km/h. Optionally, the treadmill 501 may automatically adjust the speed of the treadmill 501 to 5 km/h based on the exercise recommendation.

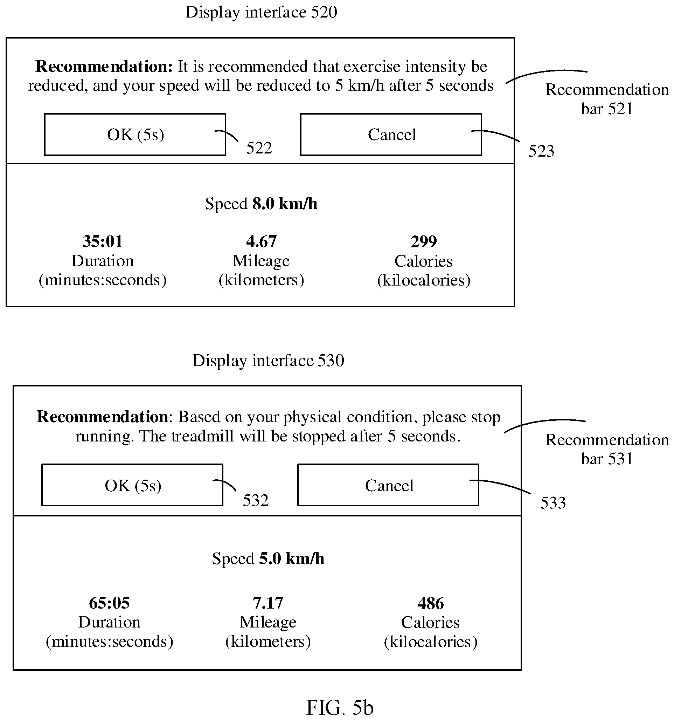

Optionally, FIG. 5b shows another display form of the display interface 520. The display interface 520 in FIG. 5b displays, in a recommendation bar 521, a determined exercise recommendation (non-taboo-type exercise recommendation) for the user: It is recommended that exercise intensity be reduced, and your speed will be reduced to 5 km/h after 5 seconds. In addition, a determining control 522 and a cancellation control 523 are provided in the recommendation bar 521. If the treadmill 501 does not receive a user operation for either of the determining control 522 and the cancellation control 523 within 5 seconds, the treadmill 501 may automatically reduce the speed to 5 km/h after 5 seconds. If the treadmill 501 receives a user operation for the determining control 522 within 5 seconds, the treadmill 501 reduces the speed to 5 km/h. If the treadmill receives a user operation for the cancellation control 523 within 5 seconds, the treadmill 501 does not reduce the speed to 5 km/h, and the treadmill 501 maintains original parameter settings.

After the user exercises on the treadmill 501 for 65 minutes, the treadmill 501 determines that the health data of the user meets the first condition (for example, a heart rate exceeds a second threshold), and a display interface 530 shown in FIG. 5a is displayed on the display of the treadmill 501. The display interface 530 displays, in a recommendation bar 511, a determined exercise recommendation (taboo-type exercise recommendation) for the user: Based on your physical condition, please stop running. Optionally, the treadmill 501 may automatically stop the treadmill 501 based on the exercise recommendation.

Optionally, FIG. 5b shows another display form of the display interface 530. A display interface 520 in FIG. 5b displays, in a recommendation bar 521, a determined exercise recommendation (taboo-type exercise recommendation) for the user: Based on your physical condition, please stop running. The treadmill will be stopped after 5 seconds. In addition, a determining control 532 and a cancellation control 533 are provided in a recommendation bar 531. If the treadmill 501 does not receive a user operation for either of the determining control 532 and the cancellation control 533 within 5 seconds, the treadmill 501 may automatically stop the treadmill after 5 seconds. If the treadmill 501 receives a user operation for the determining control 532 within 5 seconds, the treadmill 501 stops the treadmill. If the treadmill receives a user operation for the cancellation control 533 within 5 seconds, the treadmill 501 does not stop the treadmill, and the treadmill 501 maintains original parameter settings.

In the foregoing application scenario 1, the electronic device 100 may alternatively be the mobile phone 504 or the smartwatch 502. After determining an exercise recommendation, the mobile phone 504 or the smartwatch 502 sends an instruction to the treadmill 501, to indicate the treadmill 501 to output the exercise recommendation. An output manner includes but is not limited to text display, voice playback, or the like. Optionally, the mobile phone 504 or the smartwatch 502 may also output the exercise recommendation.

Application scenario 2: The user exercises in a gym.

The electronic device 100 may be a mobile phone, and the health monitoring device 101 may include an electronic device such as a smartwatch or a body fat scale. When the user exercises in the gym, the electronic device 100 and a plurality of exercise apparatuses are located in a same local area network, and can communicate with each other. The exercise apparatuses include, for example, a treadmill, an elliptical machine, a stepper, a fixed bicycle, a rowing machine, and a weightlifting apparatus.

When the user starts the weightlifting apparatus to exercise, the electronic device 100 detects that the user starts the weightlifting apparatus, and the electronic device 100 determines that the health data of the user meets the first condition (for example, the user is a patient with high blood pressure, a patient with high blood pressure is prohibited from performing an anaerobic exercise, and weightlifting is an anaerobic exercise). Because the electronic device 100 and the plurality of exercise apparatuses are in the same local area network and can communicate with each other, an exercise recommendation for the user determined by the electronic device 100 may carry location information. For example, the exercise recommendation (taboo-type exercise recommendation) for the user determined by the electronic device 100 is: Based on your physical condition, please do not lift a weight. It is recommended that you use a treadmill nearby to perform an aerobic exercise. Optionally, the weightlifting apparatus may automatically stop the weightlifting apparatus based on the exercise recommendation. Optionally, the exercise recommendation determined by the electronic device 100 may indicate a location direction of the treadmill, to help the user quickly find the treadmill.

With reference to an application scenario and an example in which the electronic device 100 is a mobile phone, the following describes an example of an implementation form of the exercise guidance method provided in this application on a display interface of the electronic device 100.

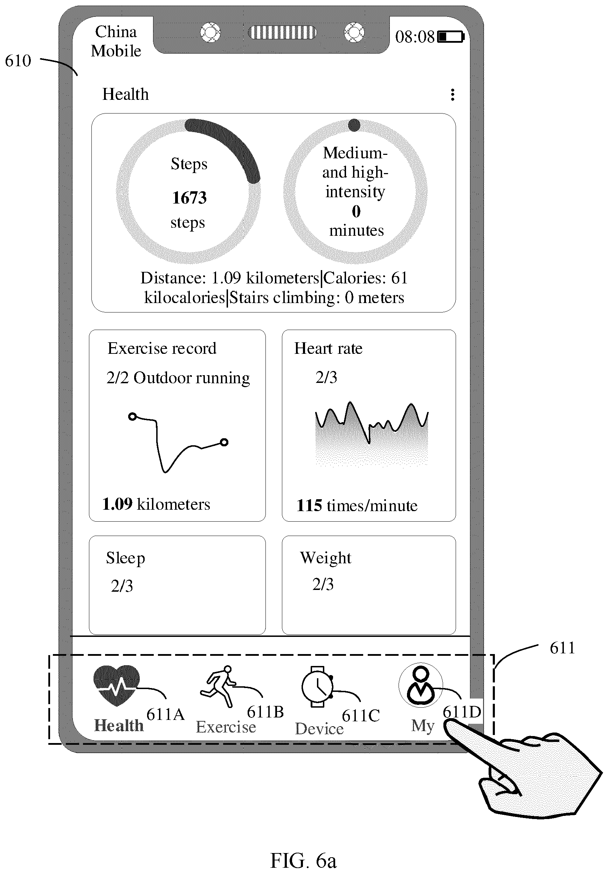

FIG. 6a to FIG. 6d show examples of display interfaces for enabling an exercise guidance function on the electronic device 100.

FIG. 6a shows an application interface 610. The application interface 610 includes exercise data obtained by the electronic device 100, for example, a quantity of steps, a distance, exercise duration, calories, a heart rate, and a weight. The application interface 610 further includes a navigation bar 611. The navigation bar 611 includes a health icon 611A, an exercise icon 611B, a device icon 611C, and a my icon 611D. It may be seen that the health icon 611A in the navigation bar 611 is marked, indicating that the current application interface 610 is an interface corresponding to the health icon 611A.

An interface corresponding to the exercise icon 611B includes various exercise types provided by the electronic device 100. The electronic device 100 may monitor data (exercise duration, an exercise distance, an exercise action, and the like) for the exercise type selected by the user.

An interface corresponding to the device icon 611C includes related information of one or more health monitoring devices 101 connected to the electronic device 100. The electronic device 100 may set and manage the health monitoring devices 101.

An interface corresponding to the my icon 611D includes personal data and exercise data that are preset by the user.

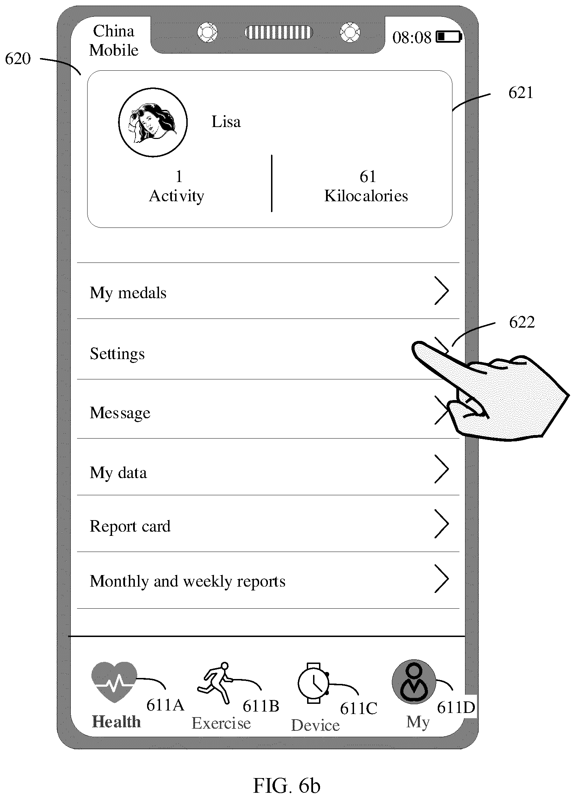

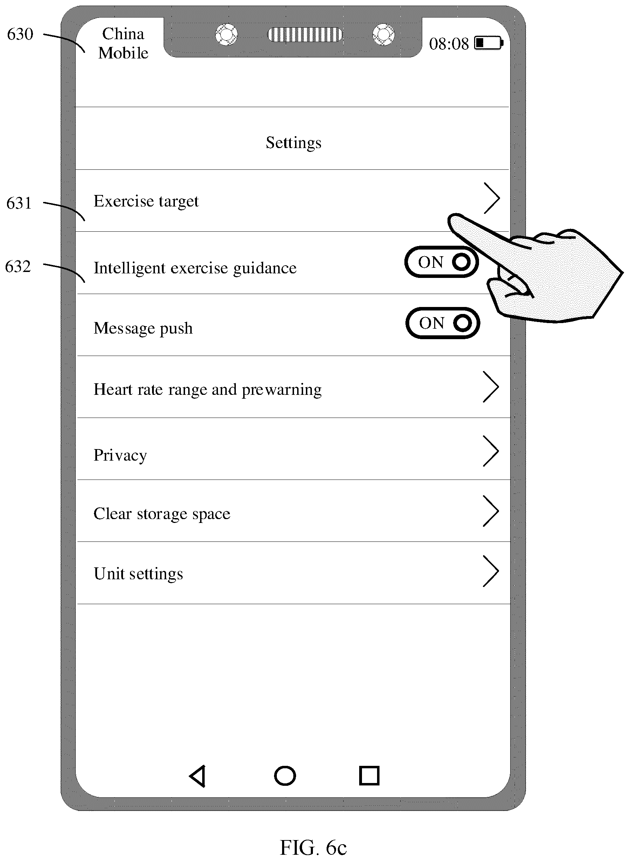

When the electronic device 100 detects a user operation for the my icon 611D, the electronic device 100 displays an application interface 620 shown in FIG. 6b in response to the operation. The application interface 620 includes a user information bar 621 and one or more option lists. The user information bar 621 indicates information about a currently login user, and the option lists include a setting option 622. When the electronic device 100 detects a user operation for the setting option 622, the electronic device 100 displays a setting interface 630 shown in FIG. 6c in response to the operation. The setting interface 630 includes one or more setting options, and the setting options include an exercise target 631, an intelligent exercise guidance 632, and the like.

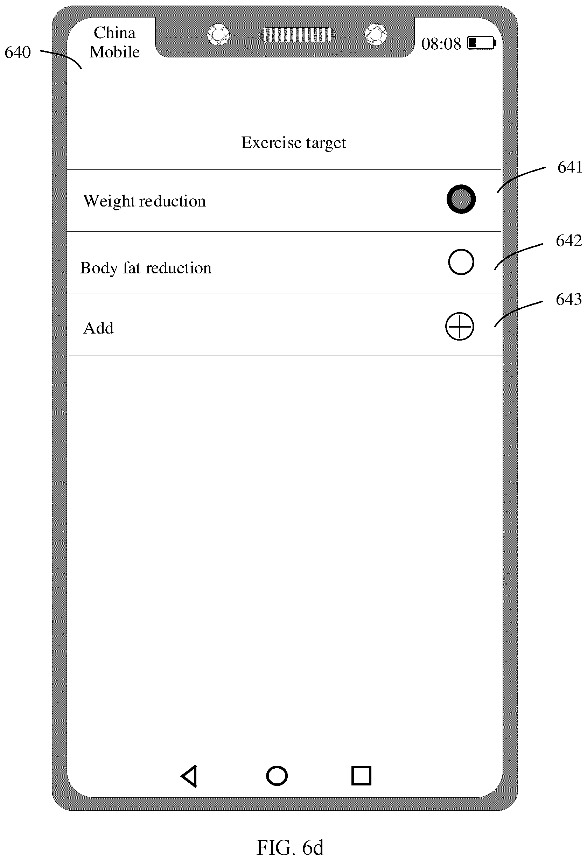

The exercise target 631 is used by the user to set an exercise target, and the electronic device 100 may provide a corresponding exercise recommendation for the user based on the exercise target set by the user. When the electronic device 100 detects a user operation for the exercise target 631, the electronic device 100 displays an application interface 640 shown in FIG. 6d in response to the operation. The application interface 640 shows examples of two exercise targets, namely, weight reduction 641 and body fat reduction 642, and further provides an addition control 643. The user may set, based on the addition control 643, an exercise target that the user intends to complete, for example, increasing maximum oxygen uptake, reducing a thigh circumference, and reducing a shank circumference.

The intelligent exercise guidance 632 is used to enable or disable an intelligent exercise guidance function. When the intelligent exercise guidance is enabled, the electronic device 100 determines an exercise recommendation based on the obtained exercise data and health data, and outputs the exercise recommendation. It can be learned that the intelligent exercise guidance 632 in FIG. 6c is in an enabled state.

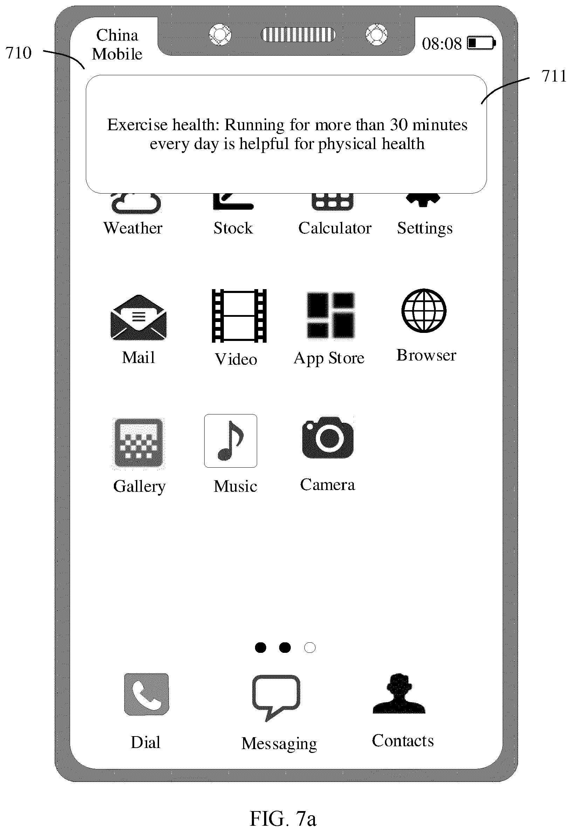

In the following, FIG. 7a to FIG. 7d show a display interface on which the electronic device 100 determines an exercise recommendation based on the obtained exercise data and health data and outputs the exercise recommendation when the intelligent exercise guidance function is enabled on the electronic device 100.

FIG. 7a shows a home interface 710. The home interface 710 includes a plurality of application icons. When the intelligent exercise guidance function is enabled on the electronic device 100, an exercise and health application may provide an exercise recommendation for the electronic device 100 in real time in a form of a notification. FIG. 7a further includes a notification bar 711. The notification bar 711 shows an exercise recommendation (non-taboo-type exercise recommendation) related to the foregoing application scenario 1: Running for more than 30 minutes every day is helpful for physical health.

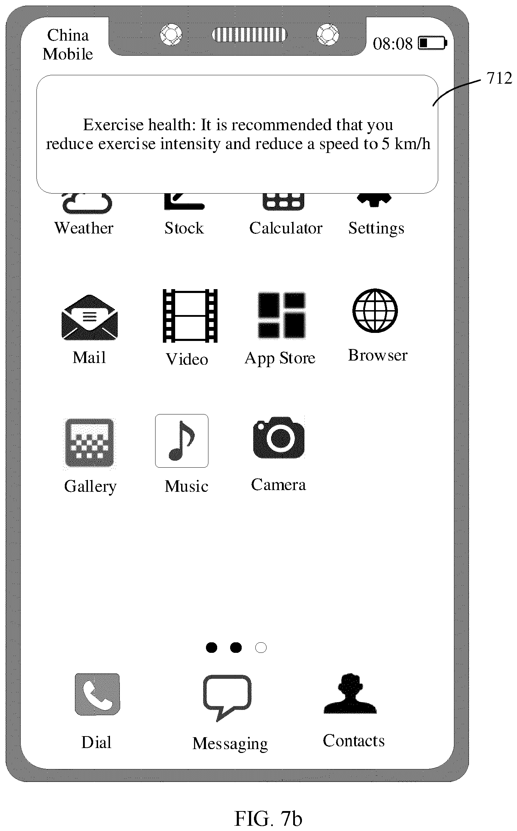

A notification bar 712 in FIG. 7b shows an exercise recommendation (non-taboo-type exercise recommendation) related to the foregoing application scenario 1: It is recommended that exercise intensity be reduced and a speed be reduced to 5 km/h.

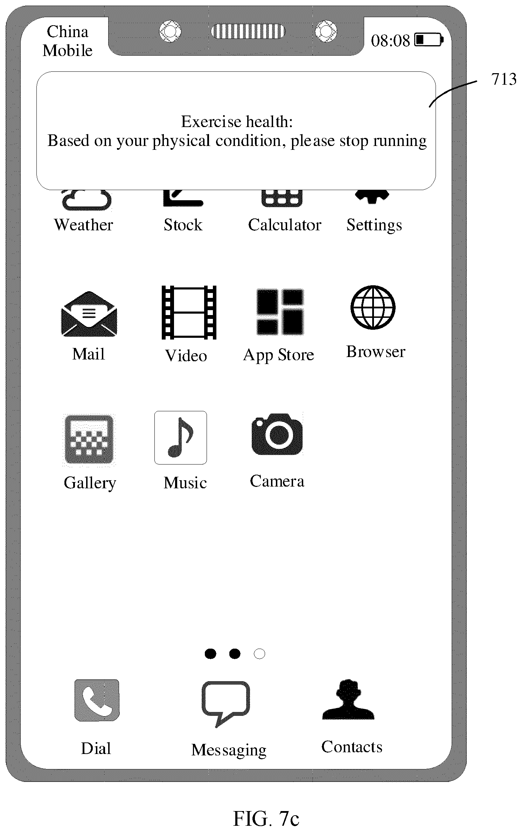

A notification bar 713 in FIG. 7c shows an exercise recommendation (taboo-type exercise recommendation) related to the foregoing application scenario 1: Based on your physical condition, please stop running.

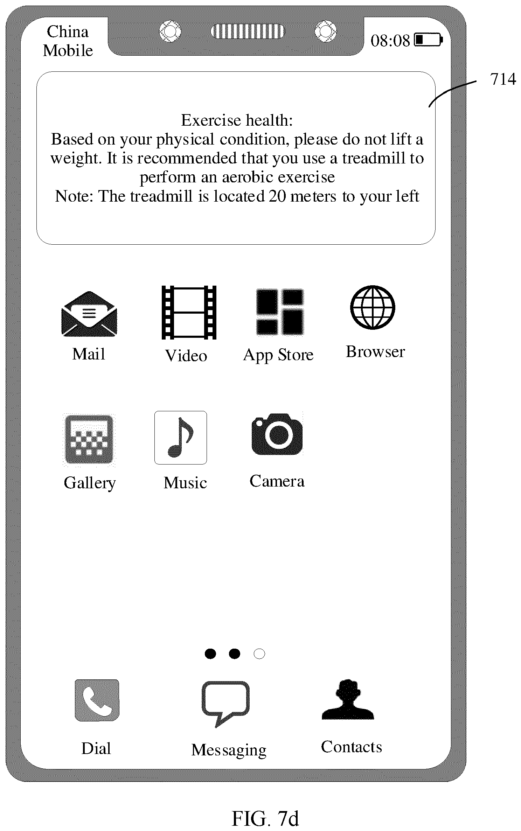

A notification bar 714 in FIG. 7d shows an exercise recommendation (taboo-type exercise recommendation) related to the foregoing application scenario 2: Based on your physical condition, please do not lift a weight. It is recommended that you use a treadmill nearby to perform an aerobic exercise. The exercise recommendation may further include location information. For example, the treadmill is located 20 meters to your left.

The exercise recommendation may be output on another device that can communicate with the electronic device 100, for example, a wearable device such as a watch or a band, an exercise apparatus, such as a treadmill or a stepper, or the like. This is not limited in this application.

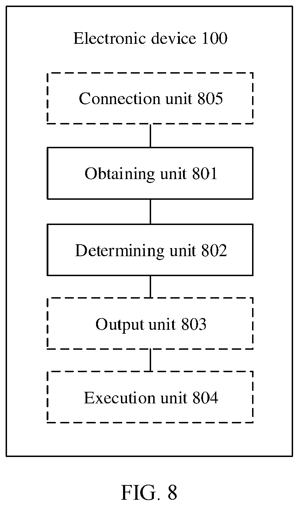

FIG. 8 is a diagram of software modules of an electronic device 100, including an obtaining unit 801 and a determining unit 802.

The obtaining unit 801 is configured to obtain first exercise data and first health data. The first exercise data indicates an exercise status or a specified exercise target.

The determining unit 802 is configured to determine a taboo-type exercise recommendation if the first health data meets a first condition. The taboo-type exercise recommendation indicates that a user is prohibited from performing an exercise corresponding to the first exercise data.

The determining unit 802 is further configured to determine a non-taboo-type exercise recommendation if the first health data meets a second condition. The non-taboo-type exercise recommendation indicates the user to adjust the exercise corresponding to the first exercise data.

In this embodiment of this application, the electronic device 100 may provide a personalized exercise recommendation for the user based on health data that is of the user in different statuses (for example, a rest state, a running state, a walking state, a swimming state, or a mountain climbing state) and that is obtained through the obtaining unit 801. In this embodiment of this application, exercise recommendations include a taboo-type exercise recommendation and a non-taboo-type exercise recommendation. The taboo-type exercise recommendation includes an exercise recommendation that the user is prohibited from performing. The non-taboo-type exercise recommendation includes an exercise recommendation that the user is recommended to perform. When the health data of the user meets different conditions, the electronic device 100 determines different exercise recommendations through the determining unit 802. The taboo-type exercise recommendation helps the user avoid an exercise that cannot be performed by the user.

In a possible implementation, the obtaining unit 801 is further configured to obtain second exercise data and second health data. The second exercise data indicates the exercise status or the specified exercise target. The determining unit 802 is further configured to determine a non-taboo-type exercise recommendation if the second health data meets a second condition. The non-taboo-type exercise recommendation indicates the user to adjust an exercise corresponding to the second exercise data. It is described herein that the electronic device 100 may continuously obtain the exercise data and the health data through the obtaining unit 801, and continuously provide corresponding exercise recommendations. If the electronic device 100 determines a taboo-type exercise recommendation when obtaining the first exercise data and the first health data, the electronic device 100 may subsequently determine, through the determining unit 802, a non-taboo-type exercise recommendation based on the obtained second exercise data and second health data.

The determining unit 802 is further configured to determine a taboo-type exercise recommendation if the second health data meets the first condition. The taboo-type exercise recommendation indicates that the user is prohibited from performing the exercise corresponding to the second exercise data. It is described herein that the electronic device 100 may continuously obtain the exercise data and the health data, and continuously provide the corresponding exercise recommendations. If the electronic device 100 determines a non-taboo-type exercise recommendation when obtaining the first exercise data and the first health data, the electronic device 100 may subsequently determine, through the determining unit 802, a taboo-type exercise recommendation based on the obtained second exercise data and second health data.

In a possible implementation, the electronic device 100 further includes an output unit 803 and an execution unit 804. The output unit 803 is configured to output, based on the taboo-type exercise recommendation after the determining unit 802 determines the taboo-type exercise recommendation, the taboo-type exercise recommendation. The execution unit 804 is configured to perform a first operation. The first operation is used to prohibit the user from performing the exercise corresponding to the first exercise data. The first operation is an operation corresponding to the taboo-type exercise recommendation. Herein, the electronic device 100 may perform, through the execution unit 804, the corresponding first operation based on the determined taboo-type exercise recommendation. The first operation is an operation that prohibits the user from performing an exercise in the taboo-type exercise recommendation. Because the taboo-type exercise recommendation is an exercise recommendation that is determined based on the health data of the user and that cannot be performed by the user, the electronic device 100 performs the taboo-type exercise recommendation through the execution unit 804. This prevents a health problem caused by an inappropriate exercise performed by the user.

The non-taboo-type exercise recommendation is similar. The electronic device 100 may perform a corresponding second operation through the execution unit 804 based on the non-taboo-type exercise recommendation determined by the determining unit 802. The second operation is used to adjust the exercise corresponding to the first exercise data.

In a possible implementation, the first exercise data is running on a treadmill. In this case, the first operation includes stopping the treadmill. An example is provided herein. To be specific, the electronic device 100 may perform the corresponding first operation based on the determined taboo-type exercise recommendation. The first exercise data is running on a treadmill. In this case, the taboo-type exercise recommendation determined by the electronic device 100 is prohibiting the user from running on the treadmill, and the electronic device 100 performs the first operation based on the taboo-type exercise recommendation, that is, stops the treadmill. Optionally, the electronic device 100 may be the treadmill or a device that establishes a communication connection to the treadmill.

In a possible implementation, the taboo-type exercise recommendation further includes exercise indication information, and the exercise indication information includes one or more of location information, direction information, apparatus information, an exercise manner, and site information for performing an exercise. To be specific, the taboo-type exercise recommendation includes both an exercise that the user is prohibited from performing and an exercise recommended to be performed, and may further provide indication information for the exercise recommended to be performed, where the indication information includes various types of information indicating the user how to perform the exercise.

The non-taboo-type exercise recommendation may also include the indication information of the exercise recommended to be performed, to indicate the user how to perform an exercise in the non-taboo-type exercise recommendation.

In a possible implementation, the electronic device 100 further includes a connection unit 805. The connection unit 805 is configured to connect to a health monitoring device before the obtaining unit 801 obtains the first exercise data and the first health data, and the health monitoring device is configured to provide the first health data and/or the first exercise data for the electronic device 100. To be specific, the health monitoring device may provide the first health data, and the first exercise data is provided by the electronic device 100; or the health monitoring device may provide the first exercise data, and the first health data is provided by the electronic device 100; or both the first health data and the first exercise data are provided by the health monitoring device.

In a possible implementation, the electronic device 100 and the health monitoring device are a same device.

In a possible implementation, the first health data includes one or more of an age, a gender, a height, a weight, a medical history, and an allergic history that are of the user and that are prestored.

In a possible implementation, the first health data is health data obtained within preset duration. Time when the electronic device 100 obtains the first health data is first time, and a time difference between current time and the first time is within the preset duration. In this case, the electronic device 100 determines an exercise recommendation based on the first health data. Optionally, if the time difference between the current time and the first time is beyond the preset duration, the electronic device 100 re-obtains latest health data as the first health data.

Optionally, time when the electronic device 100 or the health monitoring device detects the first health data is second time, and a time difference between the current time and the second time is within the preset duration. In this case, the electronic device 100 determines an exercise recommendation based on the first health data. Optionally, if the time difference between the current time and the second time is beyond the preset duration, the electronic device 100 or the health monitoring device re-detects latest health data as the first health data.

In a possible implementation, the obtaining unit 801 is further configured to obtain environment data. The environment data includes one or more of a weather condition, air humidity, air quality, a haze degree, an indoor formaldehyde concentration, and PM2.5. The first condition is related to the environment data. The determining unit 802 is further configured to determine the taboo-type exercise recommendation if the first health data and the environment data meet the first condition. It is described herein that the environment data may also be used as a determining parameter of the taboo-type exercise recommendation. For example, when an environment is poor, the electronic device 100 may determine, through the determining unit 802, a taboo-type exercise recommendation. That is, the user cannot exercise currently.

The determining unit 802 is further configured to determine the non-taboo-type exercise recommendation if the first health data and the environment data meet the second condition.

All or some of the foregoing embodiments may be implemented by using software, hardware, firmware, or any combination thereof. When software is used to implement the embodiments, all or some of the embodiments may be implemented in a form of a computer program product. The computer program product includes one or more computer instructions. When the computer program instructions are loaded and executed on a computer, all or some procedures or functions in embodiments of this application are generated. The computer may be a general-purpose computer, a dedicated computer, a computer network, or another programmable apparatus. The computer instructions may be stored in a computer-readable storage medium, or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium accessible by the computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid-state drive), or the like.

A person of ordinary skill in the art may understand that all or some of the procedures of the method in the foregoing embodiments may be implemented by a computer program by instructing related hardware. The program may be stored in a computer-readable storage medium. When the program is executed, the procedures in the foregoing method embodiments may be performed. The foregoing storage medium includes any medium that can store program code, for example, a ROM, a random access memory RAM, a magnetic disk, or an optical disc.

## Claims

1. An exercise guidance method, wherein the method comprises:

   obtaining, by an electronic device, first exercise data and first health data, wherein the first exercise data indicates an exercise status or a specified exercise target; and
   determining, by the electronic device, a taboo-type exercise recommendation if the first health data meets a first condition, wherein the taboo-type exercise recommendation indicates that a user is prohibited from performing an exercise corresponding to the first exercise data; or
   determining, by the electronic device, a non-taboo-type exercise recommendation if the first health data meets a second condition, wherein the non-taboo-type exercise recommendation indicates the user to adjust the exercise corresponding to the first exercise data.

2. The method according to claim 1, wherein the method further comprises:

   obtaining, by the electronic device, second exercise data and second health data, wherein the second exercise data indicates the exercise status or the specified exercise target; and
   determining, by the electronic device, a non-taboo-type exercise recommendation if the second health data meets the second condition, wherein the non-taboo-type exercise recommendation indicates the user to adjust an exercise corresponding to the second exercise data.

3. The method according to claim 2, wherein the method further comprises:

determining, by the electronic device, a taboo-type exercise recommendation if the second health data meets the first condition, wherein the taboo-type exercise recommendation indicates that the user is prohibited from performing the exercise corresponding to the second exercise data.

4. The method according to claim 1, wherein after the determining, by the electronic device, a taboo-type exercise recommendation, the method further comprises:
outputting, by the electronic device based on the taboo-type exercise recommendation, the taboo-type exercise recommendation, and performing, by the electronic device, a first operation, wherein the first operation is used to prohibit the user from performing the exercise corresponding to the first exercise data.

5. The method according to claim 4, wherein the first exercise data is running on a treadmill, and the first operation comprises stopping the treadmill.

6. The method according to any one of claims 1 to 5, wherein the taboo-type exercise recommendation further comprises exercise indication information, and the exercise indication information comprises one or more of location information, direction information, apparatus information, an exercise manner, and site information for performing an exercise.

7. The method according to claim 1, wherein before the obtaining, by an electronic device, first exercise data and first health data, the method further comprises:
connecting the electronic device to a health monitoring device, wherein the health monitoring device is configured to provide the first health data and/or the first exercise data for the electronic device.

8. The method according to claim 7, wherein the electronic device and the health monitoring device are a same device.

9. The method according to claim 1, wherein the first health data comprises one or more of an age, a gender, a height, a weight, a medical history, and an allergic history that are of the user and that are prestored.

10. The method according to claim 1, wherein the first health data is health data obtained within preset duration.

11. The method according to any one of claims 1 to 7, wherein the method further comprises:

obtaining, by the electronic device, environment data, wherein the environment data comprises one or more of a weather condition, air humidity, air quality, a haze degree, an indoor formaldehyde concentration, and PM2.5, and the first condition is related to the environment data; and
the determining, by the electronic device, a taboo-type exercise recommendation if the first health data meets a first condition comprises:
determining, by the electronic device, the taboo-type exercise recommendation if the first health data and the environment data meet the first condition.

12. An exercise guidance system, comprising an electronic device and a health monitoring device, wherein

the electronic device is configured to establish a connection to the health monitoring device;
the health monitoring device is configured to provide first exercise data and first health data for the electronic device, wherein the first exercise data indicates an exercise status or a specified exercise target; and
the electronic device is further configured to determine a taboo-type exercise recommendation if the first health data meets a first condition, wherein the taboo-type exercise recommendation indicates that a user is prohibited from performing an exercise corresponding to the first exercise data; or
the electronic device is further configured to determine a non-taboo-type exercise recommendation if the first health data meets a second condition, wherein the non-taboo-type exercise recommendation indicates the user to adjust the exercise corresponding to the first exercise data.

13. The system according to claim 10, wherein the electronic device is further configured to obtain second exercise data and second health data, wherein the second exercise data indicates the exercise status or the specified exercise target; and
the electronic device is further configured to determine a non-taboo-type exercise recommendation if the second health data meets the second condition, wherein the non-taboo-type exercise recommendation indicates the user to adjust an exercise corresponding to the second exercise data.

14. The system according to claim 13, wherein the electronic device is further configured to determine a taboo-type exercise recommendation if the second health data meets the first condition, wherein the taboo-type exercise recommendation indicates that the user is prohibited from performing the exercise corresponding to the second exercise data.

15. The system according to claim 11, wherein the electronic device is further configured to: after determining the taboo-type exercise recommendation, output, based on the taboo-type exercise recommendation, the taboo-type exercise recommendation, and perform a first operation, wherein the first operation is used to prohibit the user from performing the exercise corresponding to the first exercise data.

16. The system according to claim 15, wherein the first exercise data is running on a treadmill, and the first operation comprises stopping the treadmill.

17. The system according to any one of claims 11 to 16, wherein the taboo-type exercise recommendation further comprises exercise indication information, and the exercise indication information comprises one or more of location information, direction information, apparatus information, an exercise manner, and site information for performing an exercise.

18. The system according to claim 11, wherein the first health data comprises one or more of an age, a gender, a height, a weight, a medical history, and an allergic history that are of the user and that are prestored.

19. The system according to claim 11, wherein the first health data is health data obtained within preset duration.

20. The system according to claim 11, wherein the electronic device is further configured to obtain environment data, wherein the environment data comprises one or more of a weather condition, air humidity, air quality, a haze degree, an indoor formaldehyde concentration, and PM2.5, and the first condition is related to the environment data; and the electronic device is further configured to determine the taboo-type exercise recommendation if the first health data and the environment data meet the first condition.

21. The system according to claim 11, wherein the system further comprises an output device;

the electronic device is further configured to: after determining the taboo-type exercise recommendation if the first health data meets the first condition, indicate the output device to output the taboo-type exercise recommendation;
the output device is configured to output the taboo-type exercise recommendation;
the electronic device is further configured to: after determining the non-taboo-type exercise recommendation if the first health data meets the second condition, indicate the output device to output the non-taboo-type exercise recommendation; and
the output device is configured to output the non-taboo-type exercise recommendation.

22. The system according to any one of claims 11 to 21, wherein the system further comprises an execution device;

the electronic device is further configured to: after determining the taboo-type exercise recommendation if the first health data meets the first condition, indicate, based on the taboo-type exercise recommendation, the execution device to perform the first operation;
the execution device is configured to perform the first operation;
the electronic device is further configured to: after determining the non-taboo-type exercise recommendation if the first health data meets the second condition, indicate, based on the non-taboo-type exercise recommendation, the execution device to output a second operation; and
the execution device is configured to perform the second operation.

23. An electronic device, comprising one or more processors and one or more memories, wherein the memories are respectively coupled to the processors, the memories are configured to store computer program code, the computer program code comprises computer instructions, and when the computer instructions are run on the processor, the electronic device is enabled to perform the method according to claims 1 to 11.

24. A computer-readable medium, configured to store one or more programs, wherein the programs are configured to be executed by one or more processors, the programs comprise instructions, and the instructions are used to perform the method according to claims 1 to 11.

Body fat scale

Watch

...

Band

Health monitoring device 101

Electronic
device 100

FIG. 1

Electronic device 100

Antenna 1

Antenna 2

| Mobile communication module<br>2G/3G/4G/5G<br>[150] | | Wireless communication module<br>UWB/BT/WLAN/GNSS/NFC/IR/FM<br>[160] |
|---|---|---|

Speaker
[170A]

Receiver
[170B]

Microphone
[170C]

Headset jack
[170D]

Audio
module
[170]

Displays 1 to N [194]

Cameras 1 to N [193]

Indicator [192]

Motor [191]

Button [190]

Internal memory [121]

SIM card interfaces 1
to N [195]

External memory
interface [120]

Processor
[110]

Sensor module [180]

Pressure sensor
[180A]

Gyroscope sensor
[180B]

Barometric pressure
sensor [180C]

Magnetic sensor
[180D]

Acceleration sensor
[180E]

Distance sensor
[180F]

Optical proximity
sensor [180G]

Fingerprint sensor
[180H]

Temperature sensor
[180J]

Touch sensor
[180K]

Ambient light
sensor [180L]

Bone conduction
sensor [180M]

USB interface [130]

Charging
input

Charging
management
module
[140]

Power
management
module [141]

Battery [142]

FIG. 2

**Health monitoring device 101**

FIG. 3

| Health monitoring device 101 | | Electronic device 100 |

S301: Establish a connection

S302: Determine an exercise rule based on the health monitoring device 101

S303: Send monitored exercise data and health data of a user

S304: The electronic device 100 determines a taboo-type exercise recommendation when detecting that the health data of the user meets a first condition, where the taboo-type exercise recommendation indicates that the user is prohibited from performing an exercise corresponding to the exercise data of the user, and the first condition is related to the exercise rule

**S306: The electronic device 100 determines a non-taboo-type exercise recommendation when detecting that the health data of the user meets a second condition, where the non-taboo-type exercise recommendation indicates the user to adjust an exercise corresponding to the exercise data of the user, and the first condition is related to the exercise rule**

S305: The electronic device 100 performs the taboo-type exercise recommendation

S307: The electronic device 100 performs the non-taboo-type exercise recommendation

FIG. 4

Display interface 510

| Recommendation: Running for more than 30 minutes every day is helpful for physical health | | | Recommendation bar 511 |
|---|---|---|---|
| Speed **8.0 km/h** | | | |
| **00:05** | **0.00** | **1** | Exercise parameter bar 512 |
| Duration (minutes:seconds) | Mileage (kilometers) | Calories (kilocalories) | |

Display interface 520

| Recommendation: It is recommended that exercise intensity be reduced and a speed be reduced to 5 km/h | | | Recommendation bar 511 |
|---|---|---|---|
| Speed **8.0 km/h** | | | |
| **35:01** | **4.67** | **299** | Exercise parameter bar 512 |
| Duration (minutes:seconds) | Mileage (kilometers) | Calories (kilocalories) | |

Display interface 530

| Recommendation: Based on your physical condition, please stop running | | | Recommendation bar 511 |
|---|---|---|---|
| Speed **5.0 km/h** | | | |
| **65:05** | **7.17** | **486** | Exercise parameter bar 512 |
| Duration (minutes:seconds) | Mileage (kilometers) | Calories (kilocalories) | |

FIG. 5a

Display interface 520

**Recommendation:** It is recommended that exercise intensity be reduced, and your speed will be reduced to 5 km/h after 5 seconds

Recommendation bar 521

| OK (5s) | Cancel |
|---|---|

522

523

Speed **8.0 km/h**

| **35:01** | **4.67** | **299** |
|---|---|---|
| Duration | Mileage | Calories |
| (minutes:seconds) | (kilometers) | (kilocalories) |

Display interface 530

**Recommendation**: Based on your physical condition, please stop running. The treadmill will be stopped after 5 seconds.

Recommendation bar 531

| OK (5s) | Cancel |
|---|---|

532

533

Speed **5.0 km/h**

| **65:05** | **7.17** | **486** |
|---|---|---|
| Duration | Mileage | Calories |
| (minutes:seconds) | (kilometers) | (kilocalories) |

FIG. 5b

FIG. 6a

FIG. 6b

630 — China Mobile    08:08

Settings

631 — Exercise target   >

632 — Intelligent exercise guidance   ON

Message push   ON

Heart rate range and prewarning   >

Privacy   >

Clear storage space   >

Unit settings   >

FIG. 6c

FIG. 6d

FIG. 7a

China Mobile  08:08

— 712

Exercise health: It is recommended that you reduce exercise intensity and reduce a speed to 5 km/h

Weather   Stock   Calculator   Settings

Mail   Video   App Store   Browser

Gallery   Music   Camera

Dial   Messaging   Contacts

FIG. 7b

FIG. 7c

FIG. 7d

Electronic device 100

Connection unit 805

Obtaining unit 801

Determining unit 802

Output unit 803

Execution unit 804

FIG. 8

## INTERNATIONAL SEARCH REPORT

International application No.

**PCT/CN2022/106630**

### A. CLASSIFICATION OF SUBJECT MATTER

A63B 24/00(2006.01)i; G16H 20/30(2018.01)i

According to International Patent Classification (IPC) or to both national classification and IPC

### B. FIELDS SEARCHED

Minimum documentation searched (classification system followed by classification symbols)

A63B24/-,G16H20/-

Documentation searched other than minimum documentation to the extent that such documents are included in the fields searched

Electronic data base consulted during the international search (name of data base and, where practicable, search terms used)

CNABS, CNTXT, CNKI, 万方, WANFANG: 运动, 锻炼, 跑步机, 健康, 生理指标, 停止, 阈值, 建议, 环境; VEN, WPABS, USTXT, WOTXT, EPTXT, IEEE: exercise?, sport?, running machine?, health, physical sign?, stop+, threshold, suggest+, recommend+, environment.

### C. DOCUMENTS CONSIDERED TO BE RELEVANT

| Category* | Citation of document, with indication, where appropriate, of the relevant passages | Relevant to claim No. |
|---|---|---|
| Y | CN 109045629 A (QIKU INTERNET NETWORK SCIENTIFIC (SHENZHEN) CO., LTD.) 21 December 2018 (2018-12-21) description, paragraphs [0026]-[0107] | 1-24 |
| Y | CN 1968293 A (HUANG YUSHU) 23 May 2007 (2007-05-23) claim 7, and description, pages 11-33 | 1-24 |
| A | CN 109045575 A (HEFEI UNIVERSITY OF TECHNOLOGY) 21 December 2018 (2018-12-21) entire document | 1-24 |
| A | CN 111467747 A (THE FIRST AFFILIATED HOSPITAL OF FUJIAN MEDICAL UNIVERSITY et al.) 31 July 2020 (2020-07-31) entire document | 1-24 |
| A | CN 103892814 A (WUXI SHOUKANG TECHNOLOGY CO., LTD.) 02 July 2014 (2014-07-02) entire document | 1-24 |
| A | US 2016171864 A1 (SAPHIBEAT TECHNOLOGIES, INC.) 16 June 2016 (2016-06-16) entire document | 1-24 |

☐ Further documents are listed in the continuation of Box C.    ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **11 October 2022** | **26 October 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2022/106630**

| Patent document cited in search report | | | Publication date (day/month/year) | Patent family member(s) | | | Publication date (day/month/year) |
|---|---|---|---|---|---|---|---|
| CN | 109045629 | A | 21 December 2018 | None | | | |
| CN | 1968293 | A | 23 May 2007 | CN | 1968293 | B | 03 June 2015 |
| CN | 109045575 | A | 21 December 2018 | None | | | |
| CN | 111467747 | A | 31 July 2020 | CN | 212369483 | U | 19 January 2021 |
| CN | 103892814 | A | 02 July 2014 | CN | 103892814 | B | 16 February 2018 |
| US | 2016171864 | A1 | 16 June 2016 | WO | 2017100025 | A1 | 15 June 2017 |
| | | | | EP | 3387630 | A1 | 17 October 2018 |
| | | | | CN | 108369765 | A | 03 August 2018 |
| | | | | EP | 3387630 | B1 | 22 September 2021 |
| | | | | US | 9861151 | B2 | 09 January 2018 |
| | | | | EP | 3387630 | A4 | 07 August 2019 |
| | | | | CN | 108369765 | B | 11 May 2021 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110824968